# EUROPEAN PATENT APPLICATION

(11) **EP 4 473 988 A1**
(43) Date of publication of application: **11.12.2024**
(21) Application number: 23770235.2
(22) Date of filing: 16.02.2023
(51) Int. Cl.: A61M 5/24, A61M 5/31

(54) **DRUG SOLUTION ADMINISTRATION DEVICE**

(30) Priority: 14.03.2022 JP 2022038754
(71) Applicant: Terumo Kabushiki Kaisha, Tokyo 151-0072 (JP)
(72) Inventor: MATSUMOTO, Fumiya, Fujinomiya-shi, Shizuoka 418-0004 (JP)
(74) Representative: Casalonga
(86) International application number: PCT/JP2023/005355
(87) International publication number: WO 2023/176275

(57) **Abstract**

A liquid medicine administration device (12A) includes a double-ended needle (22) including first and second needle portions (112, 114), and a syringe holder (70A) that accommodates the syringe (68) and that holds the syringe (68). The syringe holder (70A) includes a holding portion (106) that holds a distal portion of the syringe (68) coaxially with a distal portion of the syringe holder (70A) by elastically biasing the distal portion of the syringe (68) radially inward.

## Description

### Technical Field

The present invention relates to a liquid medicine administration device for administering a liquid medicine to a puncture site punctured with a double-ended needle.

### Background Art

The applicant of the present application proposes, in JP 2021-159185 A, a liquid medicine administration device including a hollow double-ended needle having a distal needle portion and a rear needle portion, and a prefilled syringe connectable to the double-ended needle. In the liquid medicine administration device, the rear needle portion of the double-ended needle is disposed to face the prefilled syringe, and the double-ended needle and the prefilled syringe are disposed such that the central axis of the double-ended needle and the central axis of the prefilled syringe are substantially the same.

When the puncture operation of the liquid medicine administration device is performed, a stopper that inhibits the movement of the prefilled syringe toward the double-ended needle is removed. Thus, the prefilled syringe moves along the axial direction toward the double-ended needle by a biasing member, so that the rear needle portion of the double-ended needle punctures an end portion of the prefilled syringe. Since the rear needle portion and the prefilled syringe are connected, a liquid medicine in the prefilled syringe is discharged through the double-ended needle.

### Summary of Invention

When the above-described liquid medicine administration device is subjected to autoclave sterilization or the like, a barrel of the syringe may be deformed and curved due to a thermal load, and there is a concern that the coaxiality between the syringe and the double-ended needle may decrease due to the curvature of the barrel, and the connection between the syringe and the double-ended needle may be impaired.

An aspect of the present invention provides a liquid medicine administration device including:
a housing formed in a hollow cylindrical shape;
a syringe that includes a barrel filled with a liquid medicine and is disposed within the housing;
a syringe holder that accommodates the syringe, holds the syringe, and is disposed within the housing so as to be movable in an axial direction of the housing; and
a double-ended needle including a first needle portion that protrudes in a distal direction and punctures a puncture site, and a second needle portion that protrudes in a proximal direction, wherein
the syringe holder includes a holding portion that holds a distal portion of the syringe coaxially with a distal portion of the syringe holder by elastically biasing the distal portion of the syringe radially inward.

According to the present invention, the syringe holder includes a holding portion that can hold the distal portion of the syringe coaxially with the distal portion of the syringe holder. With this configuration, when the syringe holder and the syringe move in the distal direction within the housing, the distal end of the syringe and the double-ended needle can be reliably connected.

### Brief Description of Drawings

Fig. 1 is an external perspective view of a liquid medicine administration system according to a first configuration.
Fig. 2 is an exploded perspective view of the liquid medicine administration system illustrated in Fig. 1.
Fig. 3 is an external perspective view of a liquid medicine administration device in the liquid medicine administration system in Fig. 1.
Fig. 4 is an exploded perspective view of the liquid medicine administration device illustrated in Fig. 3.
Fig. 5 is a cross-sectional view taken along line V-V in Fig. 3.
Fig. 6 is an enlarged cross-sectional view illustrating a proximal end and its neighboring parts of the liquid medicine administration device illustrated in Fig. 5.
Fig. 7 is a cross-sectional view taken along line VII-VII in Fig. 3.
Fig. 8 is an enlarged cross-sectional view illustrating a proximal end and its neighboring parts of the liquid medicine administration device illustrated in Fig. 7.
Fig. 9 is an enlarged cross-sectional view illustrating a distal end and its neighboring parts of the liquid medicine administration device illustrated in Fig. 5.
Fig. 10 is an external perspective view of a needle cover and a syringe unit constituting the liquid medicine administration device.
Fig. 11 is an external perspective view of the syringe unit illustrated in Fig. 10.
Fig. 12 is an exploded perspective view of the syringe unit illustrated in Fig. 11.
Fig. 13 is an overall front view of the syringe unit illustrated in Fig. 12.
Fig. 14 is a plan view of a syringe holder illustrated in Fig. 13 as viewed from the proximal side.
Fig. 15 is a plan view of the syringe holder illustrated in Fig. 13 as viewed from the distal side.
Fig. 16 is an enlarged front view of a distal end and its neighboring parts of the syringe holder illustrated in Fig. 13.
Fig. 17 is a cross-sectional view taken along line XVII-XVII in Fig. 16.
Fig. 18 is a cross-sectional view taken along line XVIII-XVIII in Fig. 17.
Fig. 19 is an enlarged perspective view illustrating a distal end and its neighboring parts of a syringe unit according to a modification.
Fig. 20 is a cross-sectional view taken along line XX-XX in Fig. 19.
Fig. 21 is a plan view of a needle hub as viewed from the proximal side.
Fig. 22 is a cross-sectional view taken along line XXII-XXII in Fig. 5.
Fig. 23 is a cross-sectional view taken along line XXIII-XXIII in Fig. 5.
Fig. 24 is an exploded perspective view of a drive unit in the liquid medicine administration device.
Fig. 25 is an exploded perspective view of a locking member and a locking body in the drive unit in Fig. 24.
Fig. 26 is a cross-sectional view of the liquid medicine administration system taken along line XXVI-XXVI in Fig. 1.
Fig. 27 is a cross-sectional view taken along line XXVII-XXVII in Fig. 26.
Fig. 28 is an overall cross-sectional view illustrating a state where the skin is punctured with the needle cover of the liquid medicine administration device illustrated in Fig. 5 being pressed against the skin.
Fig. 29 is an enlarged cross-sectional view illustrating a proximal end and its neighboring parts of the liquid medicine administration device illustrated in Fig. 28.
Fig. 30 is an overall cross-sectional view illustrating another cross section of the liquid medicine administration device illustrated in Fig. 28.
Fig. 31 is an overall cross-sectional view illustrating a state in which connection between a syringe and a double-ended needle of the liquid medicine administration device is completed.
Fig. 32 is an enlarged cross-sectional view illustrating a distal end and its neighboring parts of the liquid medicine administration device illustrated in Fig. 31.
Fig. 33 is an overall cross-sectional view illustrating a state in which a gasket has moved in a barrel and the administration of a liquid medicine has been completed.
Fig. 34 is an overall cross-sectional view illustrating a case where a fixed amount of liquid medicine is administered using a plunger according to a modification.
Fig. 35 is an overall cross-sectional view illustrating a state of the liquid medicine administration device in which the double-ended needle is covered with the needle cover.
Fig. 36A is an enlarged cross-sectional view of the proximal end and its neighboring parts the liquid medicine administration device, Fig. 36A illustrating an operation of the locking member in the distal direction when the liquid medicine administration device that has finished the administration of the liquid medicine is separated from the skin, and Fig. 36B is an enlarged cross-sectional view illustrating a state in which the locking member illustrated in Fig. 36A moves to elastically deform engagement arms radially inward.
Fig. 37 is an enlarged cross-sectional view illustrating a proximal end and its neighboring parts of the liquid medicine administration device illustrated in Fig. 35.
Fig. 38 is an overall cross-sectional view illustrating another cross section of the liquid medicine administration device illustrated in Fig. 37.
Fig. 39 is an overall cross-sectional view of a liquid medicine administration system according to a second configuration.
Fig. 40 is an enlarged cross-sectional view illustrating a distal end and its neighboring parts of the liquid medicine administration device.
Fig. 41 is an enlarged cross-sectional view illustrating a puncture completion state in which a first needle portion of the liquid medicine puncture device punctures and a first antibacterial cover is folded.
Fig. 42 is an enlarged cross-sectional view illustrating a state in which first and second antibacterial covers are folded and a double-ended needle and a syringe are connected.
Fig. 43A is a schematic diagram illustrating a first sterilization device that sterilizes a needle unit by a first sterilization method, and Fig. 43B is a schematic diagram illustrating a second sterilization device that sterilizes the syringe by a second sterilization method.
Fig. 44 is an external perspective view of the liquid medicine administration device in a case where the liquid medicine administration system is sterilized by a third sterilization method by dropping sterile water into a packaging container.
Fig. 45 is an enlarged cross-sectional view of a distal end and its neighboring parts of a liquid medicine administration system according to a third configuration.
Fig. 46A is a partial cross-sectional front view of a first antibacterial cover, and Fig. 46B is a partial cross-sectional front view of a second antibacterial cover.
Fig. 47 is an enlarged cross-sectional view illustrating a puncture state in which a first needle portion of the liquid medicine administration device illustrated in Fig. 45 punctures with the first antibacterial cover being folded.
Fig. 48 is an enlarged cross-sectional view illustrating a connection completion state in which a double-ended needle and a syringe are connected with the second antibacterial cover being folded in the liquid medicine administration device illustrated in Fig. 47.
Fig. 49 is an overall cross-sectional view of a liquid medicine administration system according to a fourth configuration.
Fig. 50 is an external perspective view of a syringe holder of the liquid medicine administration device in Fig. 49.
Fig. 51 is an enlarged front view of a distal end and its neighboring parts of the syringe holder illustrated in Fig. 50.
Fig. 52 is a cross-sectional view taken along line LII-LII in Fig. 51.
Fig. 53 is a cross-sectional view taken along line LIII-LIII in Fig. 52.
Fig. 54 is a cross-sectional view taken along line LIV-LIV in Fig. 49.
Figs. 55A to 55C are enlarged operation explanatory diagrams illustrating a process for reducing a speed of the syringe unit by a deceleration mechanism with a first guide rib being inserted into a first guide groove.
Fig. 56 is an overall cross-sectional view of a liquid medicine administration system according to a fifth configuration.
Fig. 57 is an overall cross-sectional view of the liquid medicine administration device illustrated in Fig. 56.
Fig. 58 is an enlarged front view of a distal end and its neighboring parts of a syringe holder of the liquid medicine administration device illustrated in Fig. 57.
Fig. 59 is an enlarged cross-sectional view illustrating a distal end and its neighboring parts of the liquid medicine administration device illustrated in Fig. 57.
Fig. 60 is an overall cross-sectional view illustrating a puncture state of the liquid medicine administration device illustrated in Fig. 57.
Fig. 61 is an enlarged cross-sectional view illustrating a needle hub and its neighboring parts of the liquid medicine puncture device illustrated in Fig. 60.
Fig. 62 is an overall cross-sectional view illustrating a liquid medicine administration state of the liquid medicine administration device illustrated in Fig. 60.
Fig. 63 is an enlarged cross-sectional view illustrating a needle hub and its neighboring parts of the liquid medicine puncture device illustrated in Fig. 62.

### Description of Embodiments

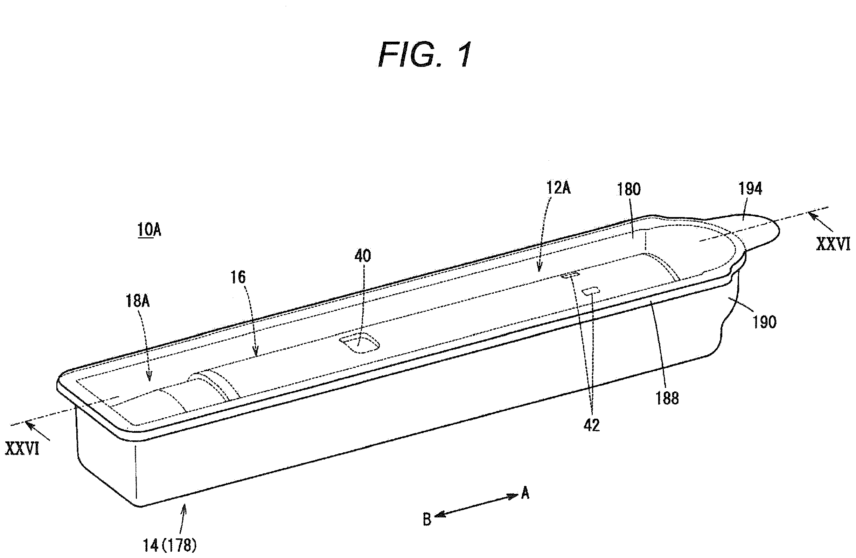

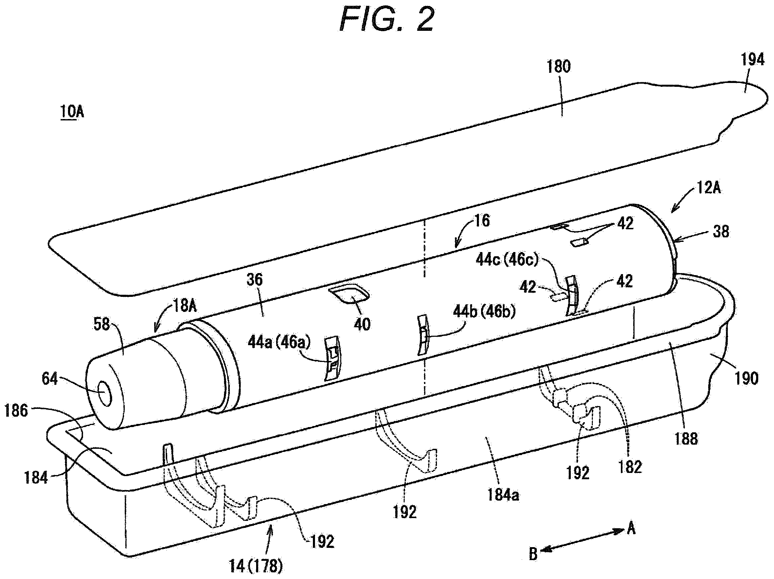

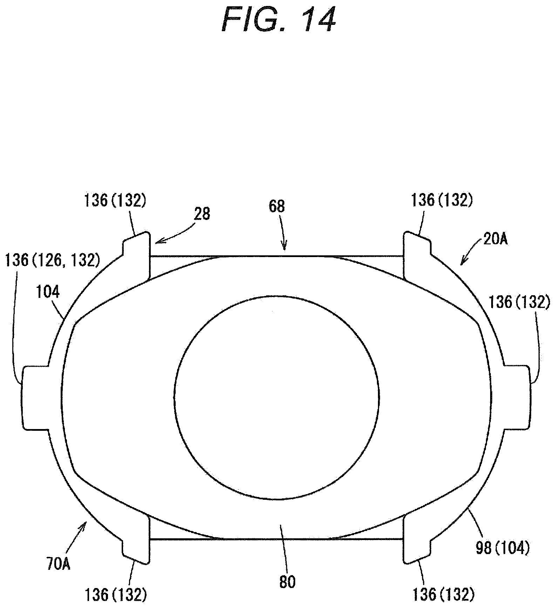

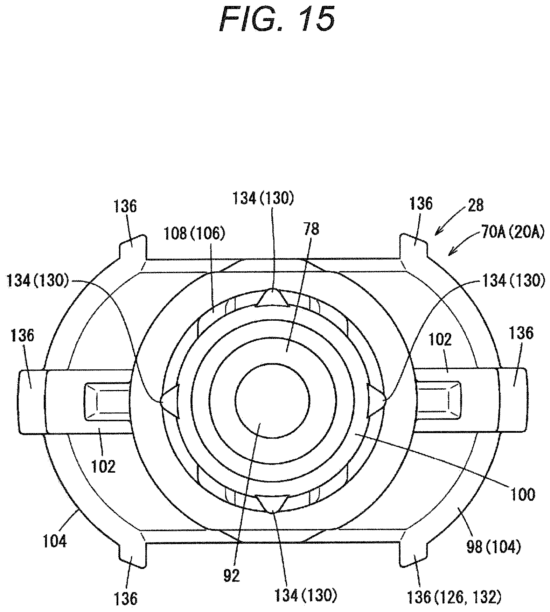

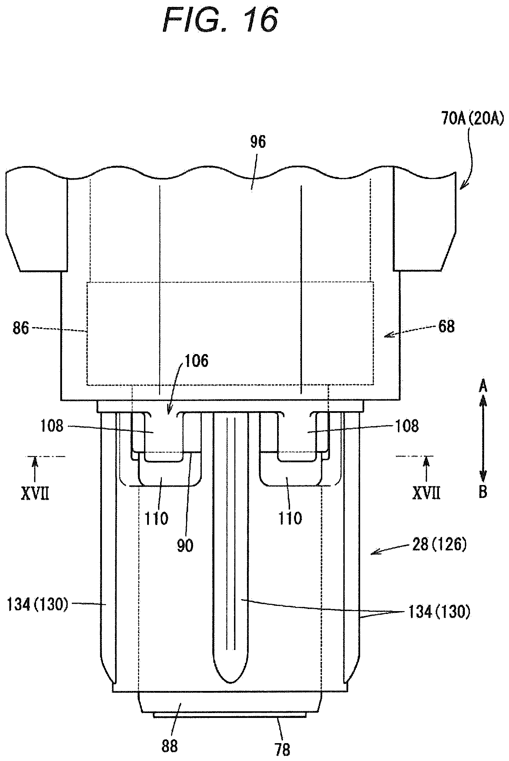

A liquid medicine administration system 10A will be described with reference to Figs. 1 to 38. As illustrated in Figs. 1 and 2, the liquid medicine administration system 10A includes a liquid medicine administration device 12A and a packaging container 14 that accommodates the liquid medicine administration device 12A.

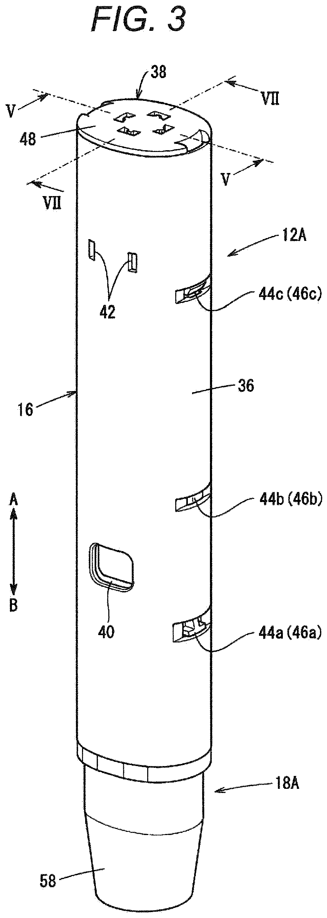

The liquid medicine administration device 12A is used, for example, for administering a liquid medicine M to a subcutaneous part (puncture site) of a patient, or a user. As illustrated in Figs. 3 to 9, the liquid medicine administration device 12A includes: a housing 16 formed in a hollow cylindrical shape; a needle cover 18A movably accommodated in the housing 16; a syringe unit 20A accommodated in the housing 16; a needle unit 26A including a double-ended needle 22 and a needle hub 24A; a guide mechanism 28 that guides the syringe unit 20A in the distal direction; and a drive unit 34 including a locking mechanism 32 that inhibits movement of the plunger 30 in the distal direction in an initial state before the user performs a puncture operation.

The housing 16 is formed of a resin material into a cylindrical shape. The cross section of the housing 16 has an elliptical shape when viewed from the axial direction (see Fig. 3). The housing 16 includes a cylinder 36 extending along the axial direction (direction of arrow A, B) and an end cap 38 that closes the proximal end of the cylinder 36.

The cylinder 36 has a predetermined length in the axial direction (direction of arrow A, B). The cylinder 36 has an opened distal end and opened proximal end. The cylinder 36 has a peripheral wall provided with a viewing window 40 and holes 42. The viewing window 40 penetrates the peripheral wall of the cylinder 36 and allows a syringe 68 accommodated in the cylinder 36 to be visually recognized from the outside.

A pair of holes 42 is formed near the proximal end of the cylinder 36 (direction of arrow A) so as to be separated from each other along the peripheral wall of the cylinder 36, and penetrates to the inside of the cylinder 36.

As illustrated in Figs. 3 to 5, the peripheral wall of the cylinder 36 includes first to third engagement holes 44a, 44b, and 44c. The first to third engagement holes 44a, 44b, and 44c are provided at positions at about 90° in the circumferential direction with respect to the opening direction of the viewing window 40. Each of the first to third engagement holes 44a, 44b, and 44c penetrates the peripheral wall of the cylinder 36.

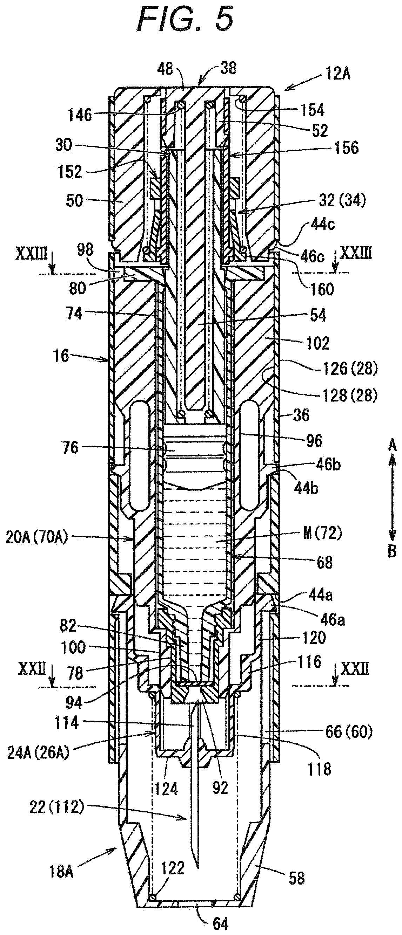

A pair of the first engagement holes 44a is located distal to (direction of arrow B) the viewing window 40 so as to be symmetrical with respect to the axial center of the cylinder 36. First engagement portions 46a of the needle hub 24A accommodated in the cylinder 36 are engaged with the first engagement holes 44a (see Fig. 5).

A pair of the second engagement holes 44b is located proximal to (direction of arrow A) the viewing window 40 so as to be symmetrical with respect to the axial center of the cylinder 36. Second engagement portions 46b of a syringe holder 70A accommodated in the cylinder 36 can be engaged with the second engagement holes 44b (see Fig. 5).

A pair of the third engagement holes 44c is located proximal to (direction of arrow A) the second engagement holes 44b so as to be symmetrical with respect to the axial center of the cylinder 36. Third engagement portions 46c of the end cap 38 attached to the proximal end of the cylinder 36 are engaged with the third engagement holes 44c (see Fig. 5).

As illustrated in Figs. 5 to 8, the end cap 38 includes a lid 48 that closes the proximal end of the cylinder 36, a retaining portion 50 that extends in the distal direction (direction of arrow B) from the outer edge of the lid 48, a holding cylindrical portion 52 that is disposed radially inside the retaining portion 50 and protrudes in the distal direction from the lid 48, and a shaft portion 54 that extends in the distal direction from the center of the lid 48. The lid 48 has a disk shape and closes the opened proximal end of the cylinder 36.

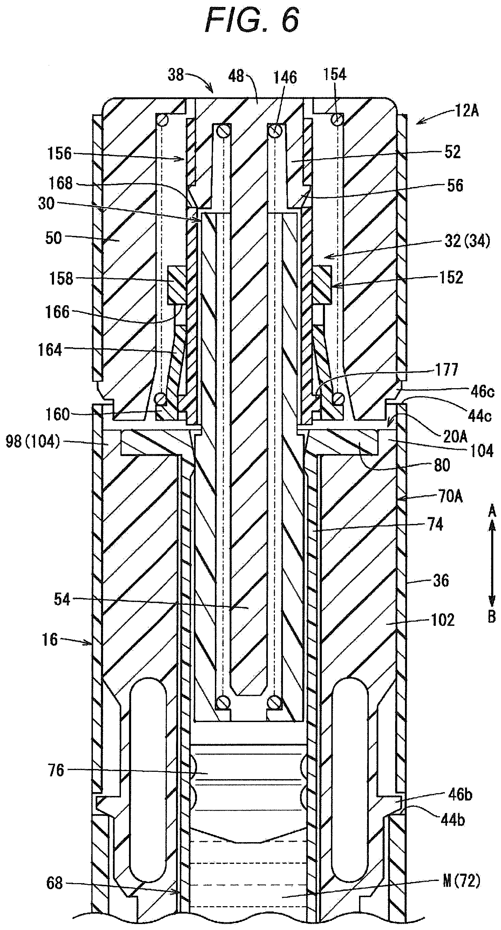

As illustrated in Fig. 6, the holding cylindrical portion 52 has a cylindrical shape opened in the distal direction, and has a plurality of engagement protrusions 56 protruding radially outward on the outer peripheral surface of the distal end. The engagement protrusions 56 are spaced apart from each other along the circumferential direction of the outer peripheral surface of the holding cylindrical portion 52. The engagement protrusions 56 are engaged with a locking body 156 described later.

The retaining portion 50 is formed in an annular shape and protrudes in the distal direction (direction of arrow B) from an end face of the lid 48 on the distal side. The retaining portion 50 has a pair of third engagement portions 46c protruding outward in the radial direction on the outer peripheral surface. The third engagement portions 46c have a protruding shape protruding from the outer peripheral surface.

The proximal end of the syringe unit 20A is in contact with the distal end of the retaining portion 50, whereby the movement of the syringe unit 20A in the proximal direction (direction of arrow A) is inhibited.

The shaft portion 54 has a shaft shape protruding in the distal direction from the end face of the lid 48 on the distal side. The shaft portion 54 is located on the axis of the housing 16. The shaft portion 54 is accommodated in the cylinder 36 and the later-described locking body 156 and syringe unit 20A, and extends to a region near the center of the cylinder 36 in the axial direction (see Fig. 5) .

The shaft portion 54 is inserted into an injection spring (biasing member) 146 and a plunger 30 constituting the drive unit 34 described later.

When the end cap 38 is attached to the proximal end of the cylinder 36, the third engagement portions 46c are engaged with the third engagement holes 44c of the housing 16, so that the end cap 38 is fixed to the housing 16, the opened proximal end is closed by the lid 48, and the shaft portion 54 is located on the axis of the cylinder 36.

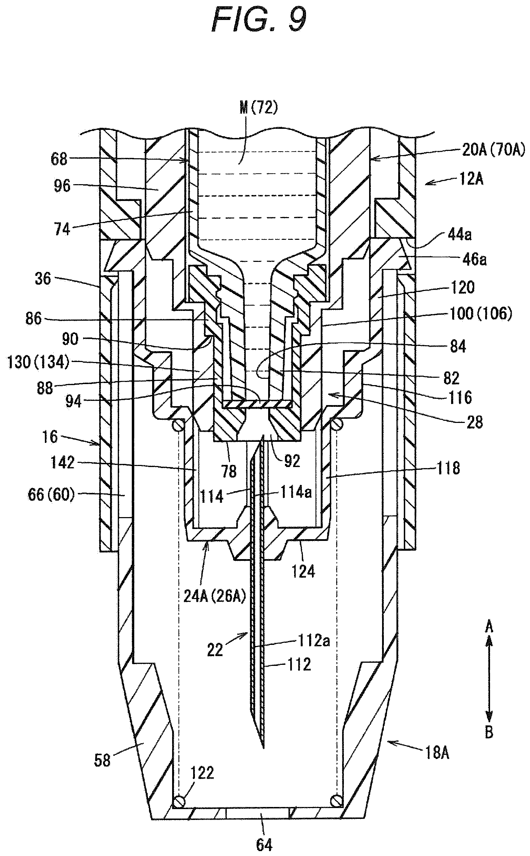

As illustrated in Figs. 5 and 9, the needle cover 18A is formed in a hollow cylindrical shape, is provided at the distal end of the housing 16, and is at least partially placed inside the cylinder 36 in the housing 16. The needle cover 18A is movable in the axial direction with respect to the housing 16. In an initial state illustrated in Figs. 5, 7, and 9 before the user performs the puncture operation, the needle cover 18A covers at least a first needle portion 112 of the needle unit 26A to be described later.

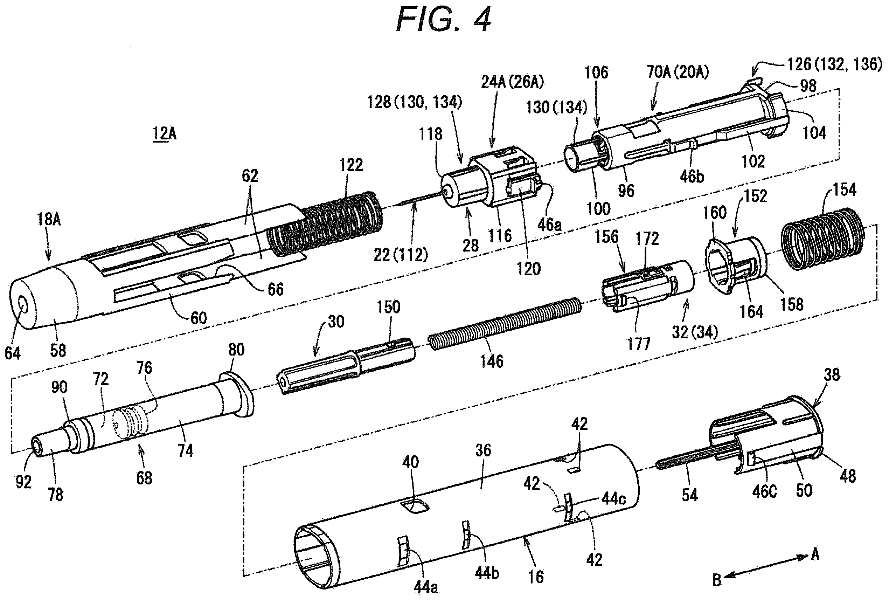

As illustrated in Figs. 4 and 5, the needle cover 18A includes a cylindrical main body 58 provided at the distal end, a cover portion 60 extending from the main body 58 in the proximal direction (direction of arrow A), and a pair of extending portions 62 extending from the proximal end of the cover portion 60.

A distal end opening 64 penetrating in the axial direction is provided at the center of the distal end of the main body 58. When the needle cover 18A is pressed against a skin S of the user in association with the puncture operation, the needle cover 18A moves in the proximal direction (direction of arrow A) relative to the housing 16, and the first needle portion 112 of the needle unit 26A is inserted into the distal end opening 64 and protrudes from the needle cover 18A in the distal direction (direction of arrow B).

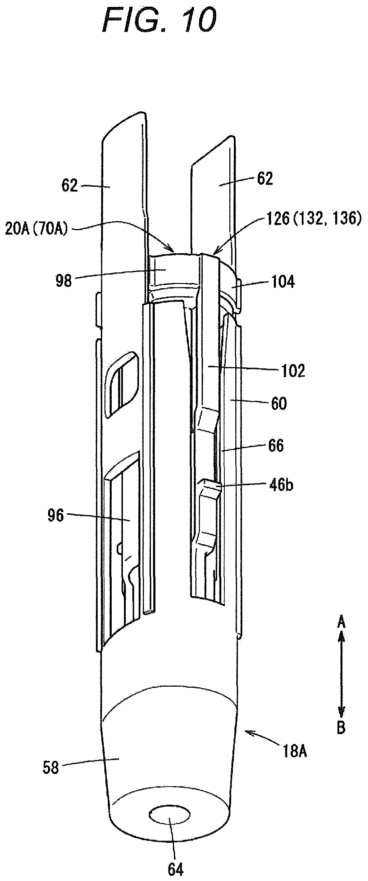

As illustrated in Figs. 4 and 10, the cover portion 60 has a pair of slits 66. The slits 66 extend in the distal direction from the proximal end of the cover portion 60 and are arranged to be symmetrical with respect to the axis of the needle cover 18A. The slits 66 are open at the proximal end of the cover portion 60. As illustrated in Fig. 10, when the syringe unit 20A is accommodated inside the needle cover 18A, a pair of holder protruding portions 102 of the syringe holder 70A can be inserted into the slits 66, respectively.

The extending portions 62 extend in the proximal direction (direction of arrow A) from the proximal end of the cover portion 60 and are disposed at positions away from the slits 66 by about 90° along the circumferential direction of the needle cover 18A. When the needle cover 18A moves in the proximal direction (direction of arrow A) relative to the housing 16, the proximal ends of the extending portions 62 come into contact with the distal end of the locking member 152 of the drive unit 34 described later to push up the locking member 152 in the proximal direction.

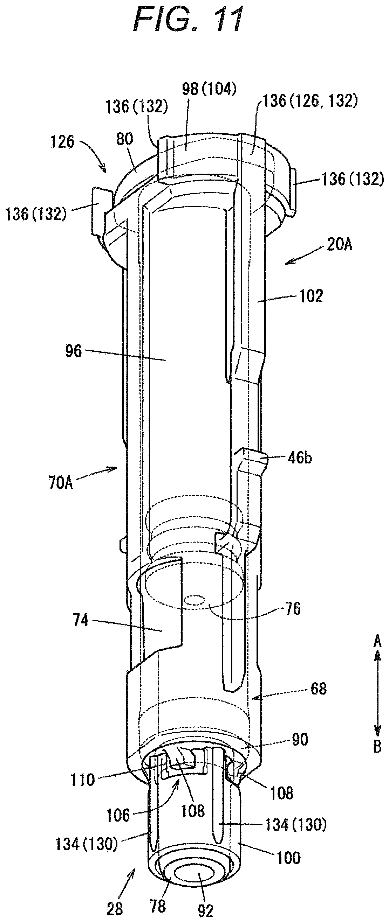

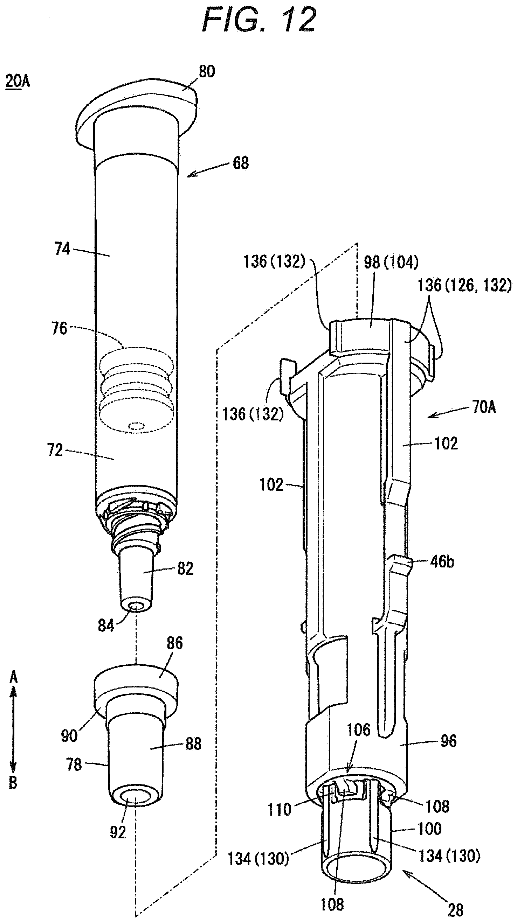

As illustrated in Figs. 11 and 12, the syringe unit 20A includes a syringe 68 and a syringe holder 70A provided on the outer periphery of the syringe 68 to hold the syringe 68. The syringe unit 20A is accommodated in the housing 16 so as to be movable along the distal direction (direction of arrow B) (see Fig. 5).

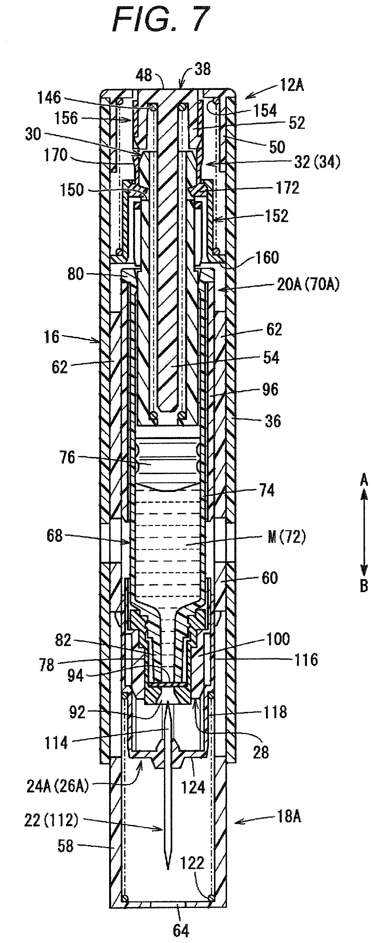

As illustrated in Figs. 5 and 7, the syringe 68 includes a barrel 74 having a medicine chamber 72 filled with the liquid medicine M, a gasket 76 capable of sliding inside the barrel 74 in a liquid-tight manner, and a cap 78 attached to a distal end of the barrel 74. The barrel 74 has, at its proximal end, a flange 80 extending radially outward from the barrel 74.

The syringe 68 includes a nozzle 82 (see Fig. 9) disposed at the distal end of the barrel 74 and having a diameter smaller than that of the barrel 74, and the cap 78 is attached to an outer peripheral surface of the nozzle 82. Thus, the outer periphery of the nozzle 82 is covered with the cap 78. A nozzle hole 84 is opened at the distal end of the nozzle 82.

As illustrated in Fig. 9, the cap 78 is formed in a cylindrical shape smaller in diameter and shorter in length in the axial direction than the syringe 68. The cap 78 includes a large-diameter portion 86 located at a proximal end and a small-diameter portion 88 located at a distal end of the large-diameter portion 86 and having a diameter smaller than that of the large-diameter portion 86, the cap 78 having a stepped portion 90 at a boundary between the large-diameter portion 86 and the small-diameter portion 88. The stepped portion 90 has a surface extending in the radial direction orthogonal to the axis of the cap 78. The cap 78 is formed to be smaller in diameter and shorter in length in the axial direction than the barrel 74 of the syringe 68, and thus, when, for example, the syringe 68 is sterilized by heat, an amount of deformation of the cap 78 due to the heat is smaller than an amount of deformation of the syringe 68.

An insertion hole 92 through which a second needle portion 114 of the double-ended needle 22 can be inserted is provided at the distal end of the small-diameter portion 88. The insertion hole 92 faces and communicates with the nozzle hole 84 of the syringe 68. The distal end of the insertion hole 92 has a trapezoidal cross-sectional shape widening in the distal direction. A lid body 94 is provided between the inner surface of the distal end of the small-diameter portion 88 and the inner surface of the distal end of the cap 78. The insertion hole 92 and the nozzle hole 84 face each other across the lid body 94. The lid body 94 is formed of an elastic material and has a disk shape having a constant thickness. The lid body 94 faces the insertion hole 92 and is held between the inner surface of the distal end of the small-diameter portion 88 and the inner surface of the distal end of the cap 78.

As illustrated in Figs. 11 and 12, the syringe holder 70A includes a holder body 96 formed in a cylindrical shape, a flange accommodating portion 98 formed at a proximal end of the holder body 96, and a holder distal cylindrical portion 100 extending in the distal direction (direction of arrow B) from a distal end of the holder body 96. The syringe holder 70A is provided to be movable in the distal direction (direction of arrow B) within the housing 16.

The holder body 96 extends along the axial direction, and the barrel 74 is accommodated inside the holder body 96. The syringe 68 may be deformed and curved by heat applied by autoclave sterilization or the like. In view of this, a clearance is formed between the holder body 96 and the barrel 74 for allowing the deformation (see Fig. 5). A pair of holder protruding portions 102 is provided on an outer peripheral surface of the holder body 96. The holder protruding portions 102 protrude radially outward from the outer peripheral surface of the holder body 96 and extend along the axial direction. The pair of holder protruding portions 102 is symmetrically arranged with respect to the axis of the syringe holder 70A.

When the syringe unit 20A is accommodated inside the needle cover 18A, the holder protruding portions 102 of the syringe holder 70A can be inserted into the slits 66, respectively (see Fig. 10).

Each of the holder protruding portions 102 includes the second engagement portion (engagement portion) 46b. The second engagement portion 46b protrudes in a direction orthogonal to the extending direction of the holder protruding portion 102. When the syringe holder 70A is accommodated in the housing 16, the second engagement portions 46b are engaged with the second engagement holes 44b of the housing 16. In an initial state before the user performs the puncture operation, the second engagement portions 46b are engaged with the second engagement holes 44b, respectively, whereby the syringe unit 20A including the syringe holder 70A is held in the housing 16 (see Figs. 5 and 6).

As illustrated in Figs. 11 to 14, the flange accommodating portion 98 is formed in a substantially elliptical shape corresponding to the flange 80 of the syringe 68 and has a recessed shape recessed from the proximal end of the syringe holder 70A. The flange 80 of the syringe 68 is accommodated inside the flange accommodating portion 98. When the flange 80 of the syringe 68 is accommodated in the flange accommodating portion 98, the syringe 68 is positioned and held in the distal direction with respect to the syringe holder 70A.

A pair of curved portions 104 protruding in the proximal direction is provided outside the flange accommodating portion 98, and the curved portions 104 constitute an outer edge of the flange accommodating portion 98 and have a curved shape protruding outward in the radial direction. A proximal support guide 140 of the guide mechanism 28 described later is disposed on the outer peripheral surfaces of the curved portions 104. The proximal ends of the holder protruding portions 102 extend to the proximal ends of the curved portions 104 and constitute a part of the proximal support guide 140.

As illustrated in Figs. 11 to 13, the holder distal cylindrical portion 100 has a cylindrical shape having a diameter smaller than that of the holder body 96. The nozzle 82 and the cap 78 of the syringe 68 are accommodated in the holder distal cylindrical portion 100. The holder distal cylindrical portion 100 is disposed so as to surround the nozzle 82 and the cap 78 of the syringe 68 (see Fig. 9). The holder distal cylindrical portion 100 is inserted into a guide cylindrical portion 118 of the needle hub 24A.

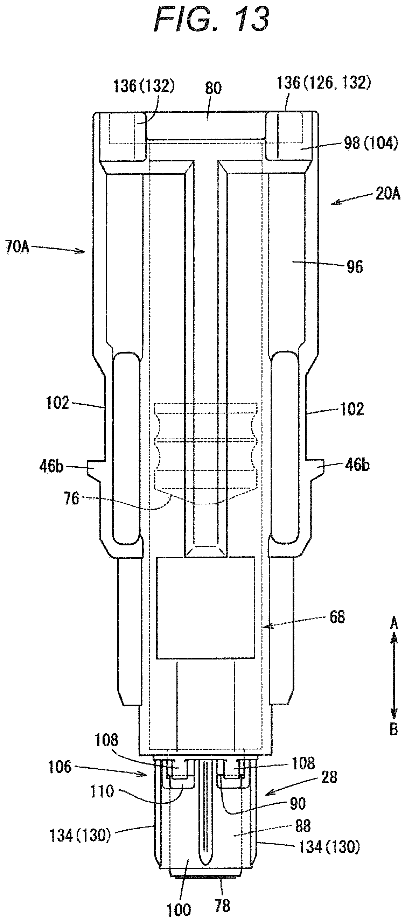

As illustrated in Figs. 13, 15, and 16, the holder distal cylindrical portion 100 includes a holding portion 106 that elastically biases the cap 78 radially inward, and a distal slide guide 130 (described later) that guides the holder distal cylindrical portion 100 during insertion into the guide cylindrical portion 118 of the needle hub 24A.

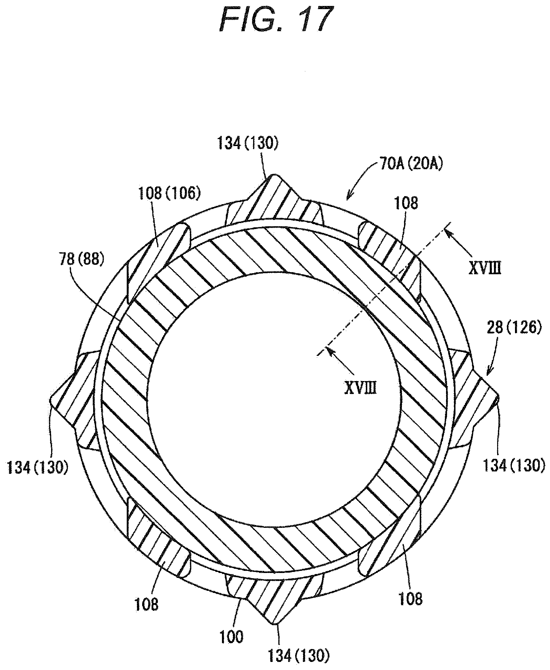

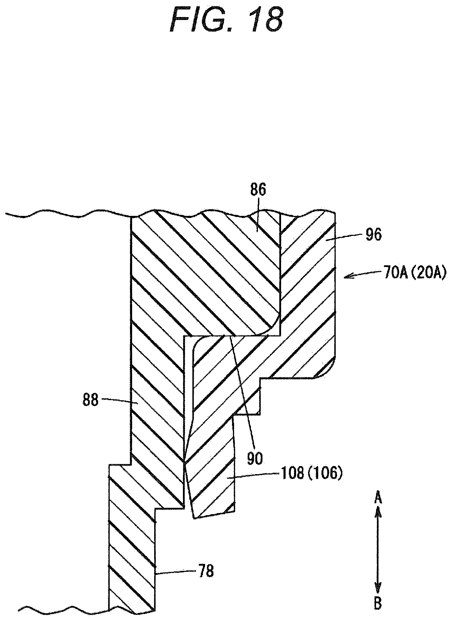

The holding portion 106 elastically biases a distal portion of the syringe 68 radially inward via the cap 78. The holding portion 106 has a plurality of flexible arms 108 that presses the outer peripheral surface of the cap 78 radially inward at a plurality of positions in the circumferential direction. The plurality of flexible arms 108 is disposed in an opening 110 opened at the proximal end of the holder distal cylindrical portion 100, and protrudes in the distal direction (direction of arrow B) from the distal end of the holder body 96 in the opening 110. The inner peripheral surfaces (radially inner ends) of the flexible arms 108 are in contact with the outer peripheral surface of the small-diameter portion 88 of the cap 78 (see Figs. 17 and 18).

The plurality of flexible arms 108 is arranged at equal intervals along the circumferential direction of the syringe holder 70A. The plurality of flexible arms 108 presses the cap 78 of the syringe 68 radially inward at a plurality of positions along the circumferential direction, thereby coaxially holding the nozzle 82 (cap 78), which is the distal portion of the syringe 68, with respect to the holder distal cylindrical portion 100 which is the distal portion of the syringe holder 70A. The number of flexible arms 108 is desirably at least three.

The cap 78 pressed by the flexible arms 108 is smaller in diameter and is shorter in length in the axial direction than the barrel 74, and thus, when heat is applied to the syringe 68 by, for example, autoclave sterilization, an amount of deformation of the cap 78 is smaller than an amount of deformation of the barrel 74 caused by the heat. Therefore, by holding the cap 78 by the flexible arms 108, the distal portion of the syringe 68 can be coaxially held with respect to the syringe holder 70A with higher accuracy via the cap 78.

As illustrated in Fig. 18, the plurality of flexible arms 108 presses the small-diameter portion 88 of the cap 78 in the radial direction.

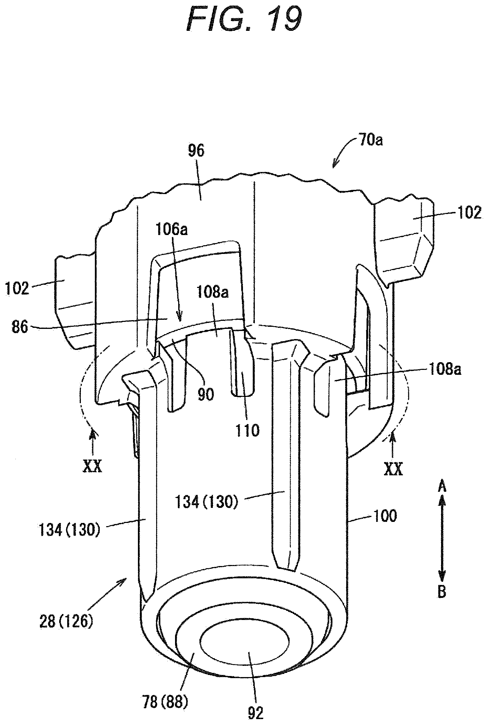

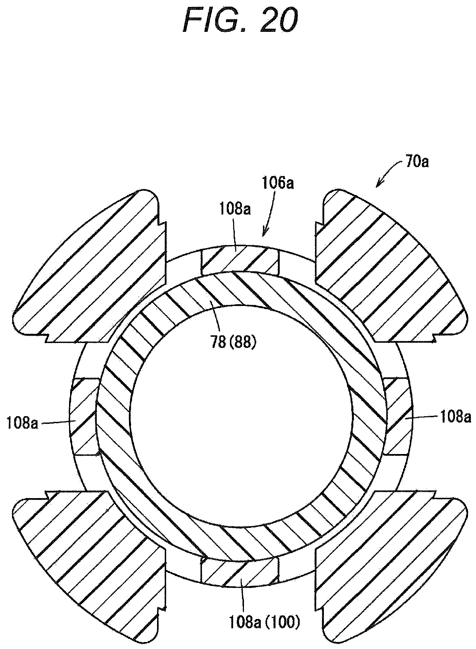

For example, a syringe holder 70a having a holding portion 106a according to a modification illustrated in Figs. 19 and 20 may be employed. The holding portion 106a of the syringe holder 70a has a plurality of flexible arms 108a that presses the outer peripheral surface of the cap 78 radially inward at a plurality of positions in the circumferential direction.

The plurality of flexible arms 108a is disposed in the opening 110 of the holder distal cylindrical portion 100, and protrudes in the proximal direction (direction of arrow A) from the distal end of the opening 110. The inner peripheral surfaces (radially inner ends) of the flexible arms 108a are in contact with the outer peripheral surface of the small-diameter portion 88 of the cap 78.

The plurality of flexible arms 108a is arranged at equal intervals along the circumferential direction of the syringe holder 70a. The plurality of flexible arms 108a presses the cap 78 of the syringe 68 radially inward, thereby coaxially holding the nozzle 82 (cap 78), which is the distal portion of the syringe 68, with respect to the holder distal cylindrical portion 100 which is the distal portion of the syringe holder 70a. The plurality of flexible arms 108a presses the small-diameter portion 88 of the cap 78 and are engaged with the distal side of the stepped portion 90 of the cap 78.

The contact area between the inner peripheral surfaces of the flexible arms 108a and the outer peripheral surface of the cap 78 is larger than the contact area between the flexible arms 108 of the syringe holder 70A and the cap 78. Therefore, the plurality of flexible arms 108a is brought into surface contact with the outer peripheral surface of the cap 78 with a larger area and presses the cap 78 radially inward, whereby the cap 78 (nozzle 82) can be coaxially held at the distal end of the syringe holder 70a.

As illustrated in Figs. 5 and 9, the needle unit 26A includes a double-ended needle 22 having first and second needle portions 112 and 114, and a needle hub 24A having a support portion 124 that supports the double-ended needle 22. The needle unit 26A is disposed distal to (direction of arrow B) the syringe unit 20A within the housing 16.

The double-ended needle 22 includes the first needle portion 112 that protrudes from the support portion 124 in the distal direction and punctures the skin S, and the second needle portion 114 protruding from the support portion 124 in the proximal direction, the double-ended needle 22 being fixed to the support portion 124 of the needle hub 24A by adhesion or the like. A lumen 112a of the first needle portion 112 communicates with a lumen 114a of the second needle portion 114.

The needle hub 24A includes a hub body 116 held by the housing 16 and a guide cylindrical portion 118 provided at the distal end of the hub body 116, and the distal end of the syringe unit 20A can be inserted into the needle hub 24A in the distal direction from the proximal end.

The hub body 116 is formed in a cylindrical shape with an open proximal end, and includes a pair of elastically tiltable arms 120 on an outer peripheral surface of the hub body 116. The distal end of each of the arms 120 is a fixed end connected to the outer peripheral surface of the hub body 116, and extends in the proximal direction from the distal end. The first engagement portion 46a protruding outward is provided at the proximal end of the arm 120 that is a movable end. When the needle hub 24A is accommodated in the housing 16 and the first engagement portions 46a are engaged with the first engagement holes 44a, the needle hub 24A is held at the distal end of the cylinder of the housing 16 (see Fig. 9).

A cover spring 122 is disposed between the hub body 116 and the needle cover 18A. The cover spring 122 is a spring member composed of a coil spring. The proximal end of the cover spring 122 is engaged with the distal end of the hub body 116. The distal end of the cover spring 122 is engaged with an inner surface of the distal end of the main body 58 of the needle cover 18A. The resilient force of the cover spring 122 biases the needle cover 18A such that the needle cover 18A relatively moves in the distal direction (direction of arrow B) with respect to the housing 16 and the needle hub 24A.

The guide cylindrical portion 118 has a bottomed cylindrical shape having the support portion 124 at the distal end and surrounds the second needle portion 114. The holder distal cylindrical portion 100 of the syringe holder 70A can be inserted into the guide cylindrical portion 118. The guide cylindrical portion 118 includes, at its distal end, the support portion 124 that supports the double-ended needle 22. The support portion 124 is orthogonal to the axial direction of the needle hub 24A, and the double-ended needle 22 is supported at the center of the support portion 124 by adhesion or the like. The first needle portion 112 is disposed outside (in the distal direction) of the guide cylindrical portion 118. That is, the first needle portion 112 and the second needle portion 114 of the double-ended needle 22 protrude in directions opposite to each other across the support portion 124.

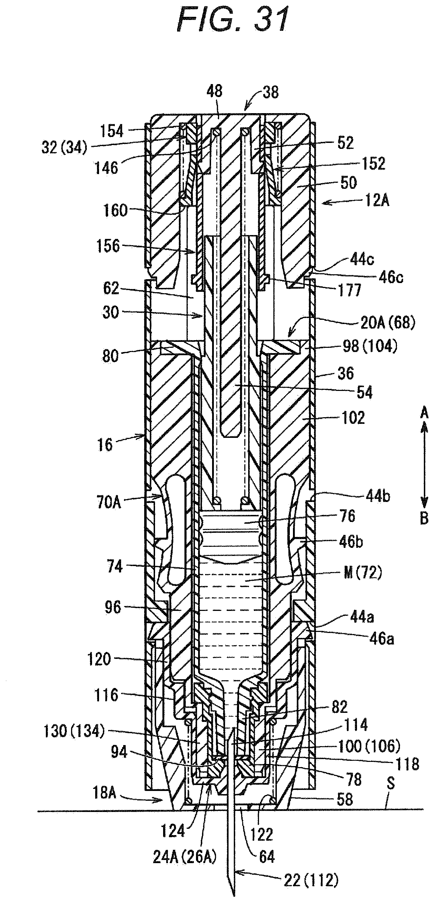

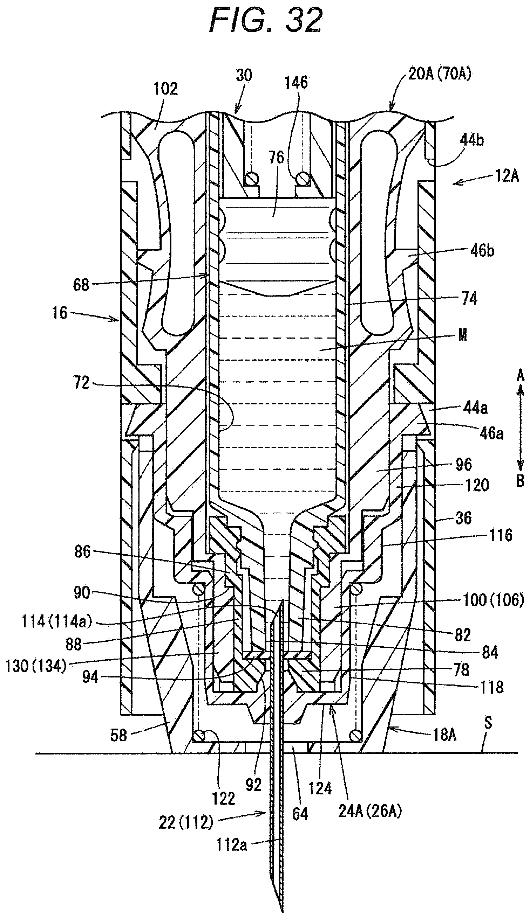

After the syringe unit 20A moves in the distal direction, the holder distal cylindrical portion 100 is inserted into the guide cylindrical portion 118, and the second needle portion 114 punctures the lid body 94, the distal end of the cap 78 held by the holder distal cylindrical portion 100 comes into contact with the support portion 124. As a result, the syringe unit 20A is retained, and the movement of the syringe unit 20A in the distal direction is stopped (see Figs. 31 and 32). The support portion 124 of the guide cylindrical portion 118 functions as a stopper capable of inhibiting the movement of the syringe unit 20A in the distal direction after the second needle portion 114 is connected to the syringe 68.

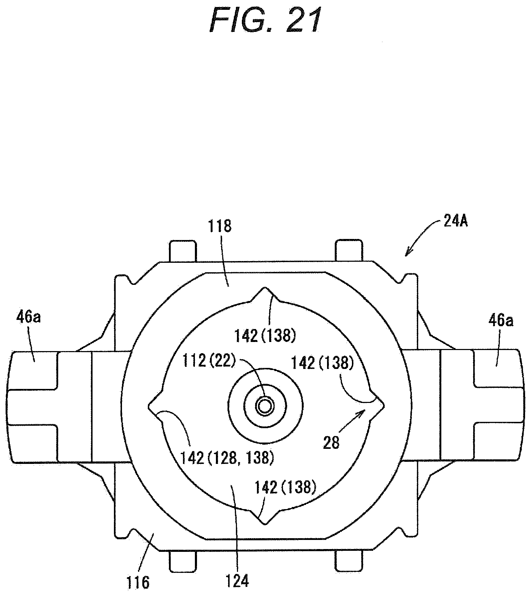

As illustrated in Fig. 21, the inner peripheral surface of the guide cylindrical portion 118 includes a distal support guide 138 of the guide mechanism 28 described later.

As illustrated in Fig. 5, the guide mechanism 28 is interposed between the housing 16 and the syringe unit 20A. The guide mechanism 28 includes a guide rib extending along the axial direction of the housing 16 and a guide groove extending along the axial direction and engaging with the guide rib. Specifically, the guide mechanism 28 includes a first guide portion 126 provided on the outer peripheral surface of the syringe holder 70A and extending along the axial direction, and a second guide portion 128 formed on the inner peripheral surfaces of the housing 16 and the needle hub 24A held by the housing 16 and extending along the axial direction, the second guide portion 128 being engaged with the first guide portion 126. A plurality of the first guide portions 126 is provided along the circumferential direction of the syringe holder 70A. A plurality of the second guide portions 128 is provided along the circumferential direction of the housing 16 and the needle hub 24A.

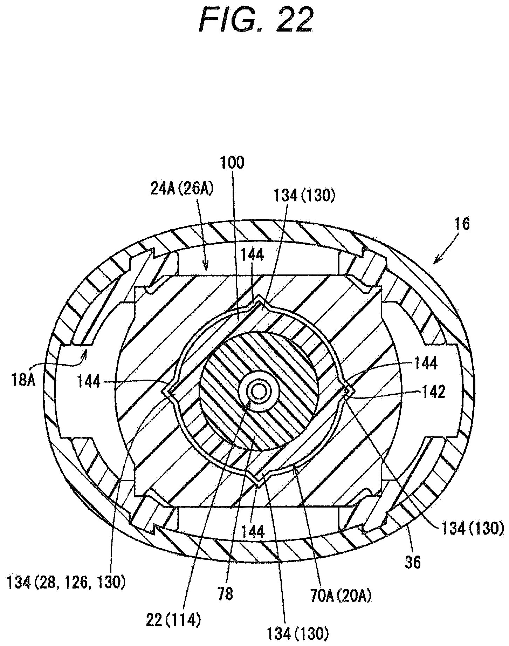

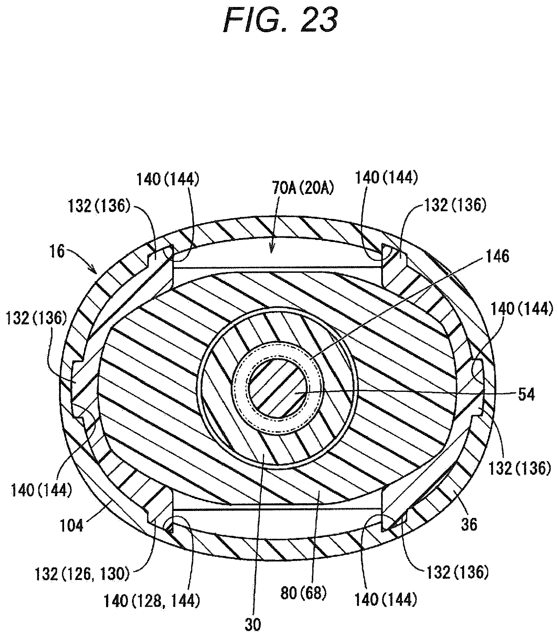

As illustrated in Fig. 11, a plurality of the first guide portions 126 is provided along the circumferential direction of the syringe holder 70A. Each of the first guide portions 126 includes a distal slide guide 130 (see Fig. 22) provided at the distal portion of the syringe holder 70A and a proximal slide guide 132 (see Fig. 23) provided at the proximal portion of the syringe holder 70A.

As illustrated in Fig. 22, the distal slide guides 130 are a plurality of first guide ribs 134 protruding radially outward from the outer peripheral surface of the holder distal cylindrical portion 100 of the syringe holder 70A. Four first guide ribs 134 are provided at equal intervals along the circumferential direction of the holder distal cylindrical portion 100. Each of the first guide ribs 134 has a triangular cross-sectional shape tapered to the outside in the radial direction as viewed in the axial direction of the syringe holder 70A.

As illustrated in Fig. 23, the proximal slide guides 132 are a plurality of second guide ribs 136 protruding radially outward from the outer peripheral surfaces of the curved portions 104 of the syringe holder 70A. Three second guide ribs 136 are provided apart from each other along the circumferential direction of each of the pair of curved portions 104. The total number of the second guide ribs 136 is six. Each of the second guide ribs 136 has a substantially rectangular cross-sectional shape as viewed in the axial direction of the syringe holder 70A.

As illustrated in Fig. 22, the second guide portion 128 includes a distal support guide 138 that is provided in the guide cylindrical portion 118 of the needle hub 24A and supports the distal slide guide 130.

The distal support guide 138 is a plurality of first guide grooves 142 recessed radially outward from the inner peripheral surface of the guide cylindrical portion 118. Four first guide grooves 142 are provided at equal intervals along the circumferential direction of the guide cylindrical portion 118. The first guide rib 134 can be inserted into the first guide groove 142. That is, the number of the first guide grooves 142 and the number of the first guide ribs 134 are the same. Each of the first guide grooves 142 has a triangular cross-sectional shape tapered to the outside in the radial direction as viewed in the axial direction of the needle hub 24A.

When the first guide rib 134 is inserted into the first guide groove 142, a slight clearance is formed between the first guide groove 142 and the first guide rib 134. The clearance between the first guide rib 134 and the first guide groove 142 is the smallest in the clearance between the syringe holder 70A and the needle hub 24A (see Fig. 22) .

When the syringe unit 20A including the syringe holder 70A moves in the distal direction along the housing 16, the distal portion of the syringe unit 20A is guided in the distal direction by the first guide ribs 134 being guided along the first guide grooves 142.

As illustrated in Fig. 23, the second guide portion 128 includes a proximal support guide 140 that is provided in the housing 16 and supports the proximal slide guide 132. The proximal support guide 140 is a plurality of second guide grooves 144 recessed radially outward from the inner peripheral surface of the cylinder of the housing 16. Six second guide grooves 144 are provided at equal intervals along the circumferential direction of the cylinder 36. The second guide rib 136 can be inserted into the second guide groove 144. That is, the number of the second guide grooves 144 and the number of the second guide ribs 136 are the same. Each of the second guide grooves 144 has a substantially rectangular cross-sectional shape as viewed in the axial direction of the housing 16.

When the syringe unit 20A including the syringe holder 70A moves in the distal direction, the proximal portion of the syringe unit 20A is guided in the distal direction by the second guide ribs 136 being guided along the second guide grooves 144. That is, the straightness of the syringe unit 20A during movement in the distal direction is enhanced by the first and second guide portions 126 and 128.

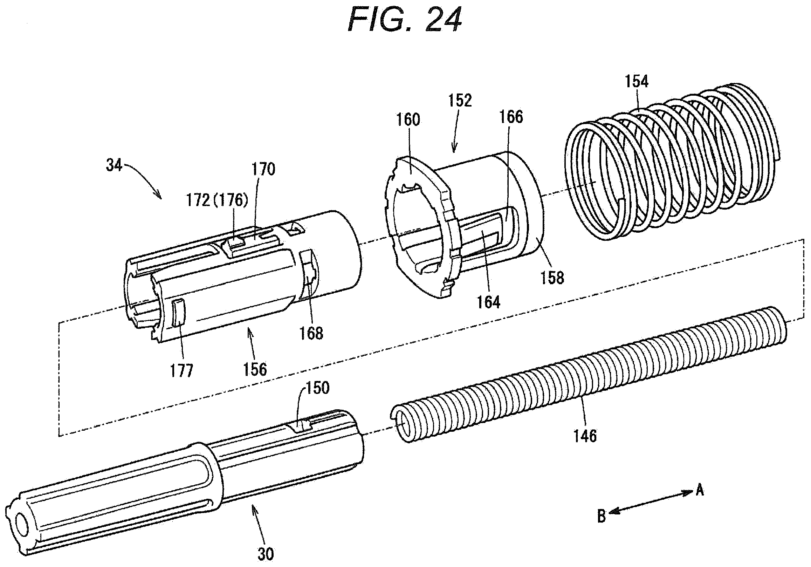

As illustrated in Figs. 6 and 24, the drive unit 34 includes a plunger 30 that is inserted into the syringe 68 and is capable of pressing the gasket 76 in the distal direction (direction of arrow B), an injection spring (biasing member) 146 that biases the plunger 30 in the distal direction, and a locking mechanism 32 that inhibits movement of the plunger 30 in the distal direction in the initial state before the user performs a puncture operation. The drive unit 34 is disposed near the proximal end of the housing 16 in the liquid medicine administration device 12A illustrated in Fig. 6.

As illustrated in Fig. 6, the plunger 30 is a cylindrical body elongated in the axial direction (direction of arrow A, B), and is disposed to be movable in the axial direction within the housing 16. The plunger 30 is formed with a substantially constant diameter along the axial direction, is located distal to (direction of arrow B) the lid 48 of the end cap 38, and is inserted into the barrel 74 of the syringe 68 on its distal side. In the initial state illustrated in Fig. 6, the distal end of the plunger 30 is disposed away from the gasket 76 by a predetermined distance in the proximal direction (direction of arrow A).

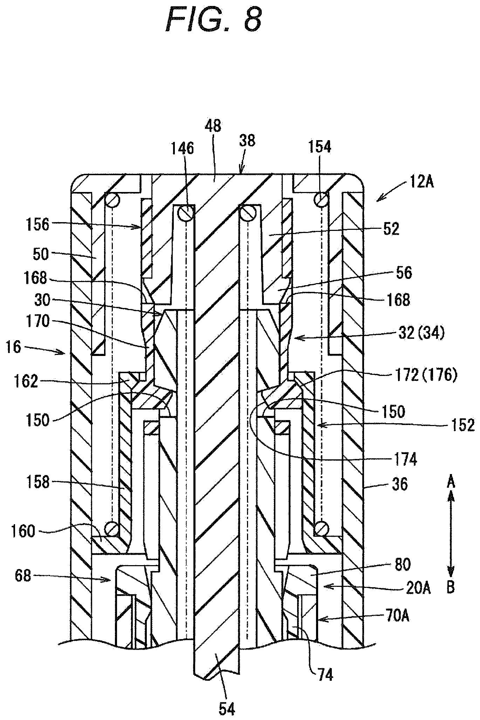

The shaft portion 54 of the end cap 38 and the injection spring 146 are inserted into the plunger 30. As illustrated in Figs. 7 and 8, a pair of locking holes 150 is provided on the outer peripheral surface of the plunger 30. The locking holes 150 are formed in the vicinity of the proximal end of the plunger 30 and penetrate the outer peripheral surface of the plunger 30 radially inward. The pair of locking holes 150 is symmetrical with respect to the axial center of the plunger 30, and locking tabs 172 of the locking body 156 of the locking mechanism 32 described later are engaged with the locking holes 150.

The injection spring 146 is a coil spring and is longer than the shaft portion 54 in the axial direction. As illustrated in Fig. 6, the injection spring 146 is interposed between the inner surface of the distal end of the plunger 30 and the end face of the lid 48 of the end cap 38, and the plunger 30 is biased in the distal direction (direction of arrow B) by resilient force of the injection spring 146.

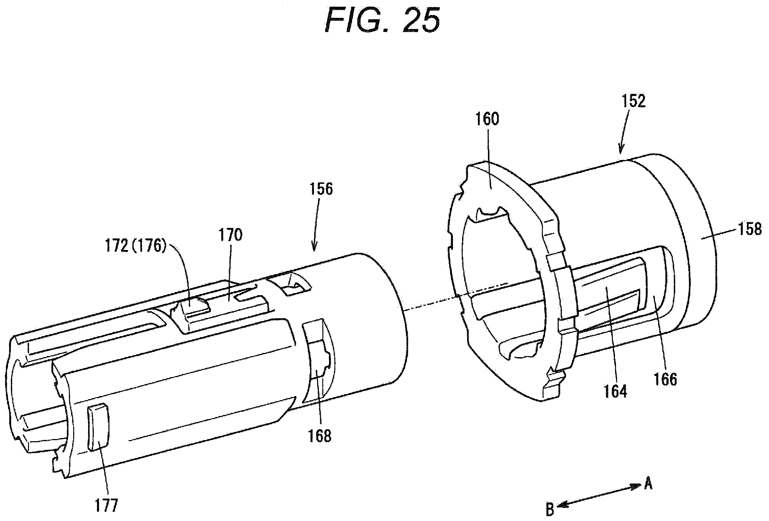

The locking mechanism 32 prevents the movement of the plunger 30 in the distal direction (direction of arrow B) in the initial state before the user performs a puncture operation. As illustrated in Figs. 6 and 25, the locking mechanism 32 includes the locking member 152 that is accommodated inside the housing 16 so as to be displaceable in the axial direction, a locking sleeve spring (elastic member) 154 that biases the locking member 152 in the distal direction, and the locking body 156 that engages with the plunger 30 in the initial state and inhibits the movement of the plunger 30 in the distal direction.

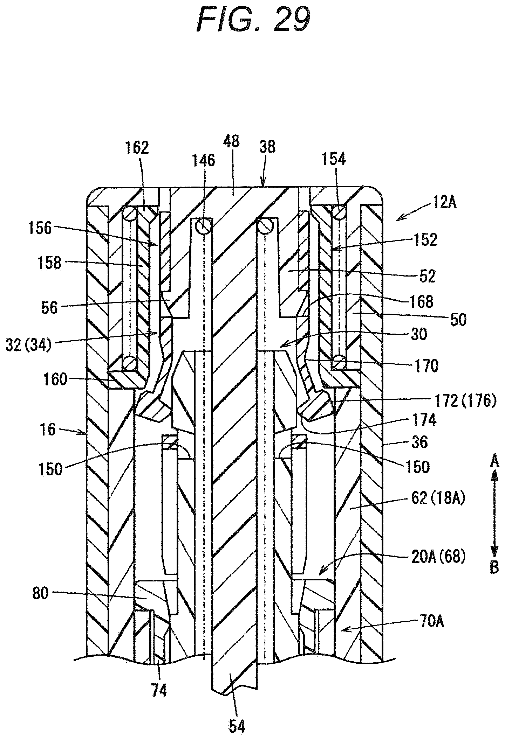

The locking member 152 is formed in a cylindrical shape surrounding the plunger 30, and is arranged on the outer periphery facing the locking holes 150 of the plunger 30 in the initial state illustrated in Fig. 8. In the puncture state, the locking member 152 is pushed up by the needle cover 18A and moved in the proximal direction to be placed on the outer periphery of the holding cylindrical portion 52 of the end cap 38 (see Fig. 29).

As illustrated in Figs. 6 and 8, the locking member 152 includes a cylindrical portion 158 and a flange portion 160 provided at a distal end of the cylindrical portion 158. The cylindrical portion 158 has, on its proximal end, an inclined portion 162 protruding radially inward from the inner peripheral surface of the cylindrical portion 158. The distal end face of the inclined portion 162 is inclined in the proximal direction (direction of arrow A) from the inner peripheral surface of the cylindrical portion 158.

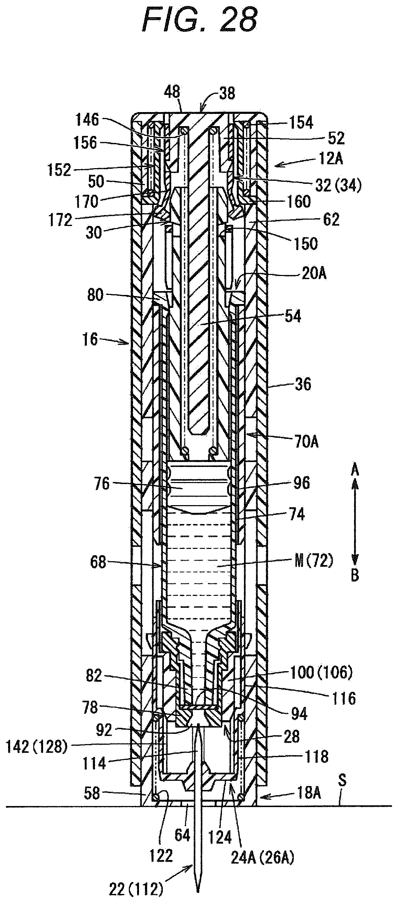

The flange portion 160 extends radially outward from the proximal end of the cylindrical portion 158. When the needle cover 18A relatively moves in the proximal direction with respect to the housing 16 at the time of puncture illustrated in Figs. 28 and 29, the proximal ends of the extending portions 62 of the needle cover 18A come in contact with the flange portion 160, so that the locking member 152 is pushed up in the proximal direction (direction of arrow A).

As illustrated in Fig. 6, the outer peripheral surface of the locking member 152 has a pair of hook portions 164 extending from the flange portion 160 in the proximal direction. Each of the hook portions 164 is formed in a corresponding retaining hole 166 opened on the outer peripheral surface of the locking member 152, and has a cantilever structure in which the distal end of the hook portion 164 is fixed to the retaining hole 166. The proximal end of the hook portion 164 is disposed inside the retaining hole 166 and can be engaged with a body projection 177 of the locking body 156 inserted into the retaining hole 166.

The locking sleeve spring 154 is a coil spring disposed on the outer periphery of the locking member 152, and is interposed between the lid 48 of the end cap 38 and the flange portion 160 of the locking member 152. The resilient force of the locking sleeve spring 154 biases the locking member 152 in the distal direction (direction of arrow B).

As illustrated in Figs. 5 to 8, the locking body 156 is disposed inside the end cap 38 within the housing 16. The locking body 156 is disposed in the vicinity of the proximal end of the plunger 30 and includes body holes 168 with which the engagement protrusions 56 of the end cap 38 are engaged, a pair of engagement arms 170 elastically deformable in the radial direction, a pair of locking tabs 172 provided at the distal ends of the engagement arms 170, and a pair of body projections 177 projecting radially outward from the outer peripheral surface.

The body holes 168 open in the outer peripheral surface of the locking body 156 and penetrate the locking body 156 radially inward. The holding cylindrical portion 52 of the end cap 38 is disposed inside the locking body 156, and the engagement protrusions 56 of the end cap 38 are engaged with the body holes 168 (see Figs. 6 and 8). Thus, the locking body 156 is fixed to the end cap 38.

As illustrated in Fig. 8, the pair of engagement arms 170 is located distal to (direction of arrow B) the body holes 168. The proximal ends of the engagement arms 170 are fixed ends, and the distal ends of the engagement arms 170 are free ends that are not fixed. The engagement arms 170 are elastically deformable with the proximal ends as supporting points. The pair of engagement arms 170 is disposed symmetrical with respect to the axial center of the locking body 156.

The locking tab 172 protrudes radially inward and radially outward at the distal end of the engagement arm 170. The locking tab 172 includes a first tab 174 protruding radially inward with respect to the distal end of the engagement arm 170 and a second tab 176 protruding radially outward with respect to the distal end.

The first tab 174 has, on its proximal end, an inclined surface inclined radially inward in the distal direction. In the initial state before the user performs the puncture operation illustrated in Fig. 8, the inclined surfaces of the first tabs 174 are engaged with the locking holes 150 of the plunger 30. At this time, the deformation of the engagement arms 170 to the outside in the radial direction is restricted due to the locking member 152 being disposed on the outer periphery of the engagement arms 170, and thus, the engagement state between the locking tabs 172 and the locking holes 150 is maintained.

That is, in the locking mechanism 32, the first tabs 174 of the locking tabs 172 are engaged with the locking holes 150 of the plunger 30, so that a lock state is established in which the movement of the plunger 30 in the distal direction (direction of arrow B) is inhibited.

During the puncture operation of the liquid medicine administration device 12A illustrated in Figs. 28 and 29 is performed, when the locking member 152 moves in the proximal direction (direction of arrow A) together with the needle cover 18A relative to the housing 16, the locking member 152 is located distal to the engagement arms 170. Therefore, the engagement arms 170 can be deformed radially outward, and thus, the locking tabs 172 of the engagement arms 170 are disengaged from the locking holes 150 of the plunger 30, and the engagement between the engagement arms 170 and the plunger 30 is released. Accordingly, the inhibition of the movement of the plunger 30 by the locking tabs 172 of the locking mechanism 32 is released.

The second tabs 176 have, on their proximal ends, inclined surfaces inclined radially outward in the distal direction. When the locking member 152 moves in the distal direction by the resilient force of the locking sleeve spring 154, the locking member 152 and the engagement arms 170 come into contact with each other, and the engagement arms 170 are elastically deformed to the inside in the radial direction. As a result, the locking member 152 is movable in the distal direction over the locking tabs 172 of the engagement arms 170. That is, the locking mechanism 32 is in an unlock state that allows the movement of the plunger 30 in the distal direction along with the movement of the needle cover 18A in the proximal direction. When the locking member 152 moves in the proximal direction and reaches the unlock position, the locking mechanism 32 enters the unlock state.

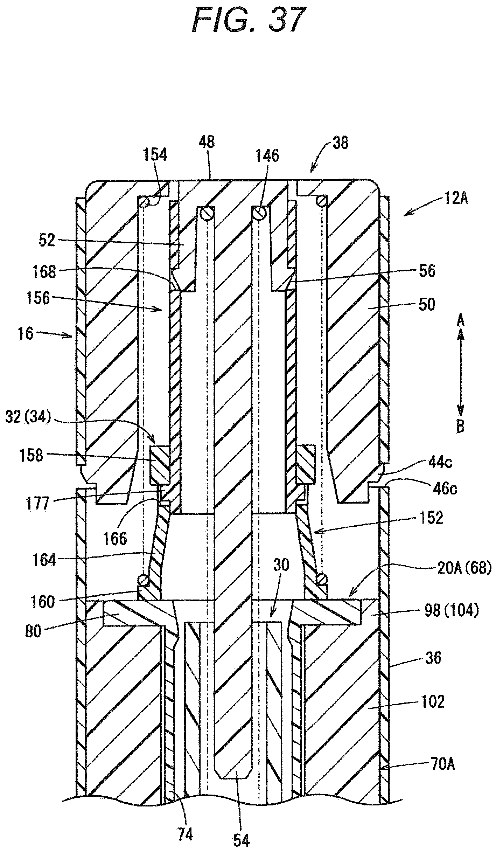

The pair of body projections 177 is formed on the outer peripheral surface of the locking body 156 at the distal end and protrudes radially outward from the outer peripheral surface. The pair of body projections 177 is disposed symmetrical with respect to the axial center of the locking body 156. As illustrated in Fig. 37, when the puncture operation is completed and the needle cover 18A and the locking member 152 move in the distal direction, the body projections 177 are inserted into the retaining holes 166 of the locking member 152 and engaged with the proximal ends of the hook portions 164. Thus, the movement of the locking member 152 in the axial direction is inhibited by the body projections 177.

The packaging container 14 illustrated in Fig. 1 accommodates the liquid medicine administration device 12A in a sealed state. As illustrated in Fig. 2, the packaging container 14 includes a container body 178 that accommodates the liquid medicine administration device 12A, a sealing film 180, and an activation inhibiting protrusion 182 capable of inhibiting activation of the drive unit 34 (see Fig. 5 and the like) when the liquid medicine administration device 12A is accommodated. The container body 178 is formed in a substantially rectangular shape in plan view. In the following, the long direction of the container body 178 is referred to as a longitudinal direction, and the short direction of the container body 178 orthogonal to the longitudinal direction is referred to as a width direction.

The container body 178 includes an accommodation recess 184 in which the liquid medicine administration device 12A is accommodated, and an opening edge 188 surrounding an opening 186 for extracting the liquid medicine administration device 12A. The container body 178 is formed of a relatively hard resin material. The container body 178 has, on its one end in the longitudinal direction, a projecting portion 190 projecting in the longitudinal direction in a semicircular shape. The opening edge 188 is formed in a rectangular shape along the opening 186 of the accommodation recess 184 and has a flange shape extending outward from the opening 186.

The accommodation recess 184 is recessed downward from the opening edge 188 formed at the upper end of the container body 178, and is formed so as to be capable of accommodating the liquid medicine administration device 12A. The accommodation recess 184 has a bottom part 184a formed in a direction opposite to the opening 186. The bottom part 184a is flat along the longitudinal direction and the width direction of the container body 178, and the liquid medicine administration device 12A is placed on a plurality of placement portions 192 protruding from the bottom part 184a.

The placement portions 192 are formed in a shape corresponding to the outer shape of the liquid medicine administration device 12A, and are spaced from each other along the longitudinal direction of the container body 178. The placement portions 192 are formed so as to connect side walls of the container body 178 in the width direction.

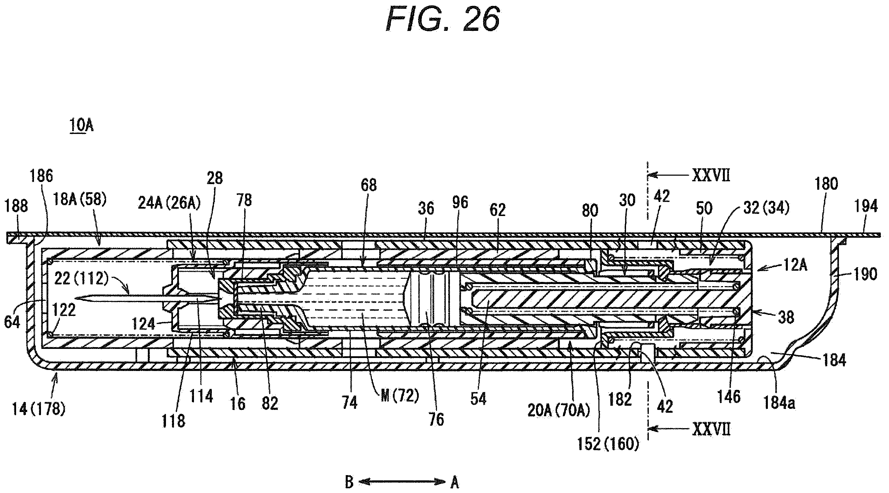

When the liquid medicine administration device 12A is accommodated in the accommodation recess 184, the liquid medicine administration device 12A is held substantially horizontally by the plurality of placement portions 192 in a state where the proximal side of the liquid medicine administration device 12A is accommodated at one end in the longitudinal direction having the projecting portion 190, and the distal side of the liquid medicine administration device 12A is accommodated at the other end in the longitudinal direction. As illustrated in Fig. 26, in a state where the liquid medicine administration device 12A is accommodated in the packaging container 14, the holes 42 of the housing 16 are positioned proximal to (direction of arrow A) the flange portion 160 of the locking member 152 of the drive unit 34.

The sealing film 180 is formed of a resin material such as polyethylene resin. The sealing film 180 has a thin sheet shape having flexibility. In plan view of the liquid medicine administration system 10A, the sealing film 180 has a rectangular shape having substantially the same dimension as the container body 178.

The sealing film 180 is placed so as to cover the opening edge 188 of the container body 178, and the outer edge of the sealing film 180 is overlaid on the opening edge 188 and heated. Thus, the outer edge of the sealing film 180 and the opening edge 188 of the container body 178 are bonded (fused). The liquid medicine administration device 12A is accommodated in the container body 178 (accommodation recess 184) in a sealed state (see Figs. 1 and 26) by the container body 178 and the sealing film 180.

The sealing film 180 has a grip portion 194 extending in the longitudinal direction at one end thereof in the longitudinal direction. When the sealing film 180 is fused to the container body 178, the grip portion 194 protrudes outward (in the longitudinal direction) from the container body 178.

When extracting the liquid medicine administration device 12A from the packaging container 14, the user grips the grip portion 194, pulls up the sealing film 180 upward from the container body 178, and peels off the sealing film 180 from the opening edge 188 of the container body 178 to open the container body 178.

As illustrated in Fig. 2, the activation inhibiting protrusion 182 is provided integrally with the placement portion 192 disposed closest to one end side in the longitudinal direction. The activation inhibiting protrusion 182 is provided on the bottom part 184a of the accommodation recess 184 together with the placement portion 192, protrudes from the bottom part 184a toward the opening 186, and protrudes further to the opening 186 than the placement portion 192. A pair of the activation inhibiting protrusions 182 is disposed apart from each other in the width direction of the container body 178.

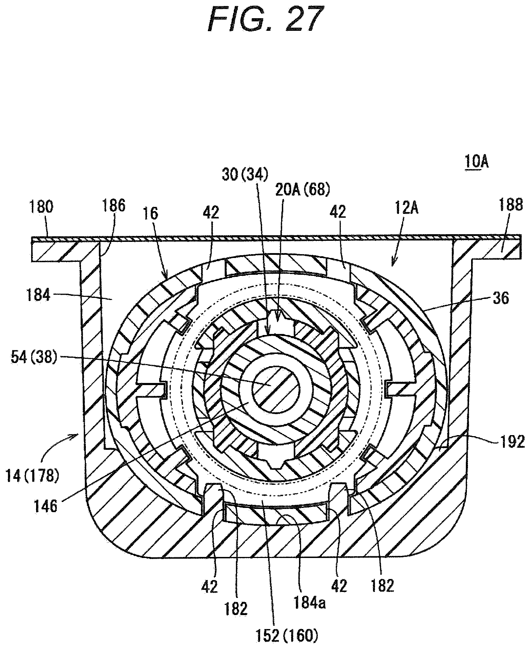

As illustrated in Fig. 27, when the liquid medicine administration device 12A is accommodated in the accommodation recess 184 and placed on the placement portions 192, ends of the activation inhibiting protrusions 182 are inserted into the housing 16 through the holes 42 of the housing 16.

As illustrated in Fig. 26, in a state where the liquid medicine administration device 12A is accommodated in the packaging container 14, the liquid medicine administration device 12A is in an initial state before the puncture operation is performed. In the initial state of the liquid medicine administration device 12A, the activation inhibiting protrusions 182 are placed proximal to (direction of arrow A) the flange portion 160 of the locking member 152 of the drive unit 34. In other words, the activation inhibiting protrusions 182 are located between an initial position where the locking member 152 is positioned in the distal direction and an unlock position where the needle cover 18A relatively moves in the proximal direction with respect to the housing 16 and the locking member 152 moves in the proximal direction.

In a state where the liquid medicine administration device 12A is accommodated in the packaging container 14, the activation inhibiting protrusions 182 are inserted into the housing 16 through the holes 42 to inhibit the transition of the locking member 152 to the unlock position. That is, in a state where the liquid medicine administration device 12A is accommodated in the packaging container 14, the activation inhibiting protrusions 182 restrict the movement of the locking member 152 in the proximal direction to inhibit the activation of the drive unit 34. When the liquid medicine administration device 12A is extracted from the packaging container 14, the activation inhibiting protrusions 182 are withdrawn from the housing 16, so that the inhibition of activation of the drive unit 34 is released.

Next, a case where, in the liquid medicine administration system 10A, the liquid medicine administration device 12A is extracted from the packaging container 14 and the puncture operation is performed will be described.

As described above, in a state where the liquid medicine administration device 12A is accommodated in the packaging container 14, the activation inhibiting protrusions 182 are inserted into the housing 16 through the holes 42 and are located proximal to the flange portion 160 of the locking member 152. Therefore, the movement of the locking member 152 in the proximal direction is restricted.

For this reason, when the liquid medicine administration system 10A which is unopened is accidentally dropped and receives an external force, the movement of the locking member 152 to the unlock position is prevented by the activation inhibiting protrusions 182, and the activation of the drive unit 34 accompanying the movement of the locking member 152 in the proximal direction is inhibited.

In the liquid medicine administration system 10A illustrated in Fig. 1, the user grips the grip portion 194 of the sealing film 180 and peels the sealing film 180 upward from the container body 178 to open the container body 178.

The user inserts his/her finger into the projecting portion 190 through the opening 186 of the container body 178 that has been opened, and extracts the liquid medicine administration device 12A from the proximal side of the liquid medicine administration device 12A to the outside of the packaging container 14 through the opening 186 (see Fig. 2). At this time, the activation inhibiting protrusions 182 are removed from the holes 42 of the housing 16, so that the inhibition of activation of the drive unit 34 by the activation inhibiting protrusions 182 is released. That is, the drive unit 34 is shifted from an activation inhibition state to an activation release state by removal of the activation inhibiting protrusions 182. The activation inhibiting protrusions 182 are provided on the bottom part 184a of the accommodation recess 184 and protrude toward the opening 186. Therefore, the activation inhibiting protrusions 182 are easily and reliably removed from the inside of the housing 16 by moving and extracting the liquid medicine administration device 12A to the outside from the opening 186.

The liquid medicine administration device 12A extracted from the packaging container 14 is in an initial state which is a state before puncture illustrated in Figs. 5 and 7. In the initial state of the liquid medicine administration device 12A, the locking tabs 172 of the engagement arms 170 of the locking body 156 are inserted into the locking holes 150 of the plunger 30 from the radially outer side, and the engagement arms 170 are prevented from tilting (elastically deforming) radially outward by the locking member 152 biased in the distal direction by the locking sleeve spring 154. That is, the engagement arms 170 cannot be elastically deformed radially outward. Therefore, the liquid medicine administration device 12A is in a lock state in which the movement of the plunger 30 in the distal direction is inhibited by the locking mechanism 32 of the drive unit 34.

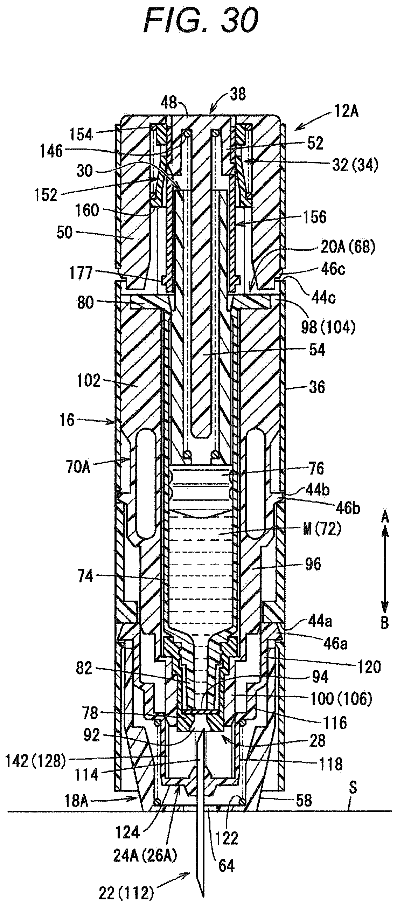

The user holds the housing 16 of the liquid medicine administration device 12A and presses the distal end of the needle cover 18A protruding from the distal end of the housing 16 against a desired puncture site of the skin S in such a manner that the distal end of the needle cover 18A is substantially perpendicular to the skin S. The user further pushes the housing 16 continuously toward the skin S side (distal side, direction of arrow B), and thus, as illustrated in Figs. 28 and 30, the needle cover 18A is pushed by the skin S, and the needle cover 18A relatively moves in the proximal direction (direction of arrow A) with respect to the housing 16 against the elastic force of the cover spring 122.

As illustrated in Figs. 28 and 30, the first needle portion 112 of the double-ended needle 22 protrudes from the distal end opening 64 of the needle cover 18A toward the distal side (direction of arrow B) along with the relative movement of the needle cover 18A in the proximal direction. Accordingly, the liquid medicine administration device 12A transitions to a puncture state where the first needle portion 112 of the double-ended needle 22 punctures the skin S and is inserted to a predetermined depth.

As illustrated in Figs. 28 and 29, with the movement of the needle cover 18A in the proximal direction (direction of arrow A), the proximal ends of the extending portions 62 of the needle cover 18A come into contact with and push up the flange portion 160 of the locking member 152 in the proximal direction (direction of arrow A). The locking member 152 moves in the proximal direction while compressing the locking sleeve spring 154 by the needle cover 18A. When the locking member 152 reaches the unlock position where the locking member 152 is positioned proximal to the engagement arms 170 having the locking tabs 172, the engagement arms 170 can be elastically deformed outward.

The plunger 30 of the drive unit 34 is biased in the distal direction (direction of arrow B) by the resilient force of the injection spring 146, whereby the locking tabs 172 move outward with the engagement arms 170 being tilted along the locking holes 150. Thus, the locking tabs 172 are disengaged from the locking holes 150 (see Fig. 29).

Accordingly, the lock state of the plunger 30 by the locking tabs 172 constituting the locking mechanism 32 is released, and the plunger 30 starts moving in the distal direction (direction of arrow B) by the resilient force of the injection spring 146. That is, the needle cover 18A functions as an activation switch for activating the drive unit 34 (plunger 30) that has been inhibited from being activated in the initial state before the puncture operation is performed by the user.

When the plunger 30 starts to move in the distal direction, the distal end of the plunger 30 comes in contact with the proximal end of the gasket 76. Thereafter, the gasket 76 is pushed in the distal direction by the plunger 30, and the plunger 30 and the gasket 76 move integrally. At this time, since the medicine chamber 72 on the distal side of the gasket 76 is filled with the liquid medicine M and the distal hole (nozzle hole 84) of the barrel 74 is closed by the lid body 94, the liquid medicine M is not discharged from the barrel 74.

Therefore, the gasket 76 does not move in the distal direction by the liquid medicine M filled in the barrel 74, and the plunger 30 moves in the distal direction, so that the syringe unit 20A including the syringe 68 is pushed in the distal direction via the gasket 76. Thus, the second engagement portions 46b of the syringe holder 70A are disengaged from the second engagement holes 44b of the housing 16, and the holding state of the syringe holder 70A by the housing 16 is released (see Fig. 31).

The syringe unit 20A pushed by the plunger 30 starts to move in the distal direction along the housing 16. At this time, the second guide ribs 136 of the syringe holder 70A move in the distal direction along the second guide grooves 144, so that the syringe unit 20A including the syringe holder 70A is guided in the distal direction (see Fig. 23).

As the plunger 30 moves in the distal direction, the syringe unit 20A moves in the distal direction, and the holder distal cylindrical portion 100 of the syringe holder 70A is inserted into the guide cylindrical portion 118 of the needle unit 26A via the hub body 116. The plurality of first guide ribs 134 provided on the holder distal cylindrical portion 100 is respectively inserted into the first guide grooves 142 of the guide cylindrical portion 118 as illustrated in Fig. 22, so that the distal portion of the syringe unit 20A is guided in the distal direction along the needle hub 24A.

That is, due to the configuration in which the first guide portions 126 are provided on the outer peripheral surface of the syringe holder 70A, the second guide portions 128 are provided on the inner peripheral surfaces of the needle hub 24A and the housing 16, and the first guide portions 126 are engaged with the second guide portions 128, the inclination of the syringe unit 20A with respect to the housing 16 is appropriately prevented when the syringe unit 20A moves in the distal direction along the housing 16, whereby the straightness of the syringe unit 20A during movement in the distal direction can be enhanced.

As the syringe unit 20A further moves in the distal direction, the distal end of the syringe unit 20A approaches the second needle portion 114 of the double-ended needle 22, and the second needle portion 114 starts to puncture the lid body 94. As illustrated in Figs. 31 and 32, the proximal end of the second needle portion 114 penetrates the lid body 94 and is inserted into the barrel 74 through the nozzle hole 84. As a result, the medicine chamber 72 of the syringe 68 and the double-ended needle 22 are connected via the second needle portion 114, and the medicine chamber 72 communicates with the subcutaneous part of the user through the lumens 112a and 114a of the double-ended needle 22.

After the second needle portion 114 of the double-ended needle 22 and the distal end of the syringe 68 are connected, the syringe unit 20A further moves in the distal direction, so that the distal end of the cap 78 held by the holder distal cylindrical portion 100 of the syringe holder 70A comes into contact with the support portion 124 of the needle hub 24A (see Figs. 31 and 32). Thus, the movement of the syringe unit 20A in the distal direction is inhibited, so that the syringe unit 20A stops at a predetermined position, and the second needle portion 114 is further inserted deeply into the barrel 74.
Accordingly, a connection completion state is established where the connection between the double-ended needle 22 and the syringe 68 is completed.

After the syringe unit 20A stops moving in the distal direction, the gasket 76 is continuously pushed in the distal direction by the plunger 30, and the sealed state of the medicine chamber 72 is released by the connection with the double-ended needle 22. Thus, the gasket 76 pushed by the distal end of the plunger 30 can move in the distal direction in the medicine chamber 72. The gasket 76 is pushed by the plunger 30 and moves in the distal direction along the barrel 74, so that the gasket 76 expels the liquid medicine M in the medicine chamber 72 in the distal direction. As a result, the liquid medicine M is discharged from the lumen 114a of the second needle portion 114 of the double-ended needle 22 through the lumen 112a of the first needle portion 112, and the administration of the liquid medicine M to the subcutaneous part of the user is started.

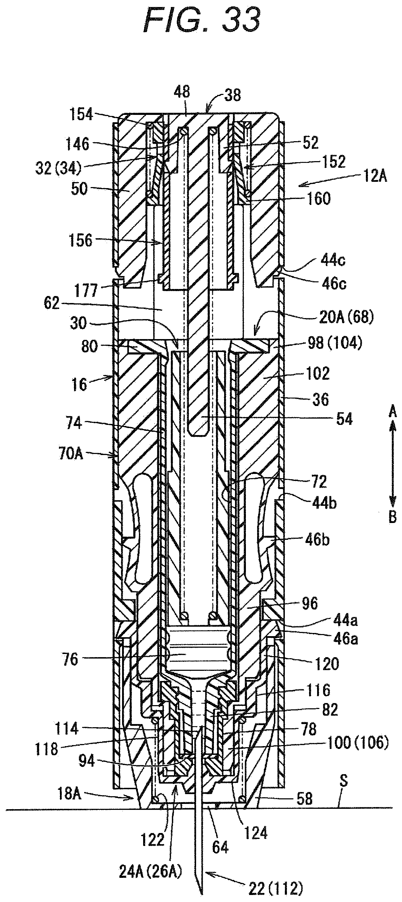

As illustrated in Fig. 33, when the gasket 76 pushed by the plunger 30 reaches the distal end of the medicine chamber 72, the administration of all the liquid medicine M filled in the medicine chamber 72 is completed.

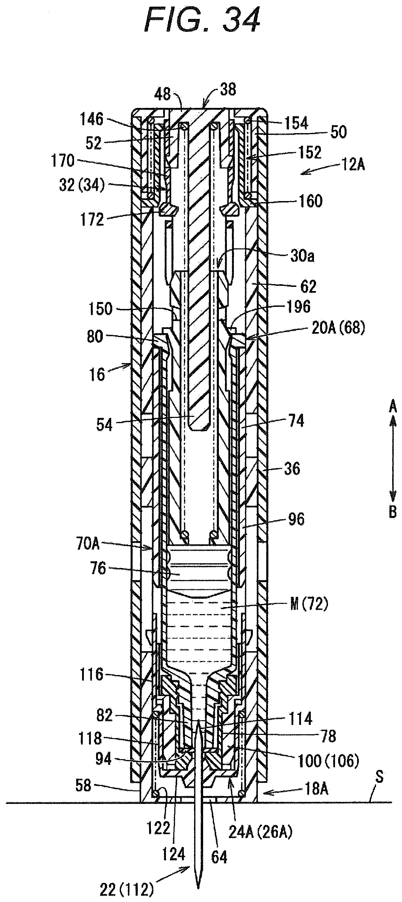

For example, a plunger 30a according to a modification illustrated in Fig. 34 may be employed. The plunger 30 has, on its outer peripheral surface, a pair of projections 196 protruding outward in the radial direction. The projections 196 are disposed in the vicinity of the proximal end of the plunger 30a and protrude in a direction orthogonal to the axial direction of the plunger 30a. The pair of projections 196 is disposed symmetrical with respect to the axial center of the plunger 30a.

When the second needle portion 114 of the double-ended needle 22 is connected to the distal portion of the syringe 68 and the plunger 30a pushes the gasket 76 in the distal direction to administer the liquid medicine M, the liquid medicine M in the medicine chamber 72 starts to be administered through the double-ended needle 22 by the movement of the gasket 76. The plunger 30a moves and the projections 196 come into contact with the flange 80 of the syringe 68, so that the movement of the plunger 30a in the distal direction is stopped.

As a result, as illustrated in Fig. 34, the movement of the plunger 30a in the distal direction is stopped by the projections 196 before the gasket 76 reaches the distal end of the medicine chamber 72, so that only a portion of the liquid medicine M filled in the medicine chamber 72 can be administered. Therefore, it is possible to administer only a predetermined amount of the liquid medicine M in the medicine chamber 72.

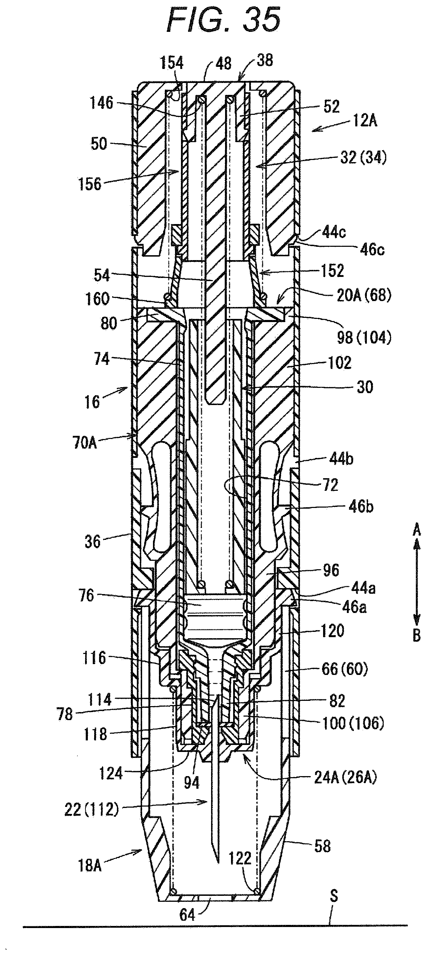

After the administration of the liquid medicine M illustrated in Fig. 33 is completed, the user releases the pressing of the liquid medicine administration device 12A against the skin S. Thus, the needle cover 18A is biased in the distal direction (direction of arrow B) by the resilient force of the cover spring 122. As illustrated in Fig. 35, the distal end of the needle cover 18A protrudes from the distal end of the housing 16, and the distal end of the needle cover 18A moves to a position distal to the first needle portion 112 of the double-ended needle 22. Accordingly, the first needle portion 112 of the double-ended needle 22 is completely covered by the needle cover 18A. The first engagement portions 46a of the needle cover 18A are engaged with the first engagement holes 44a of the housing 16.

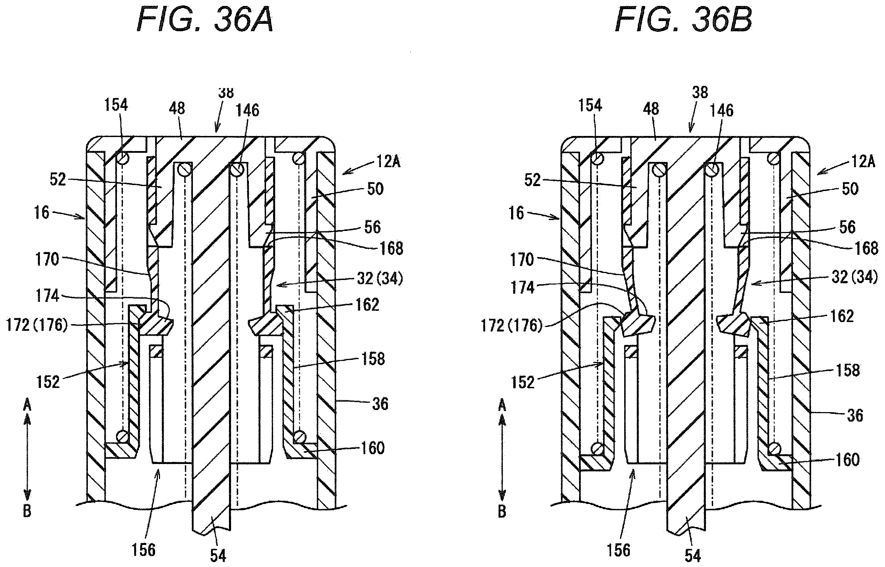

With the movement of the needle cover 18A in the distal direction, the biasing of the locking member 152 by the needle cover 18A in the proximal direction is released, and thus, the locking member 152 moves in the distal direction (direction of arrow B) by the resilient force of the locking sleeve spring 154. As illustrated in Fig. 36A, the inclined portions 162 come in contact with the second tabs 176 of the locking tabs 172 as the locking member 152 moves in the distal direction. At this time, the plunger 30 moves in the distal direction, so that the plunger 30 is not located inside the locking tabs 172.

Therefore, when the locking member 152 further moves in the distal direction as illustrated in Fig. 36B, the locking tabs 172 are pushed inward by the inclined portions 162, the engagement arms 170 are elastically deformed inward, and the inclined portions 162 move in the distal direction over the locking tabs 172. That is, the locking member 152 is movable in the distal direction with respect to the locking body 156.

As illustrated in Figs. 35 and 37, when the locking member 152 moves in the distal direction with respect to the locking body 156, the body projections 177 of the locking body 156 are inserted into the retaining holes 166 of the locking member 152, and the body projections 177 are retained by the proximal ends of the hook portions 164 and the retaining holes 166. Thus, the locking member 152 is held by the locking body 156 and is prevented from moving in the axial direction.

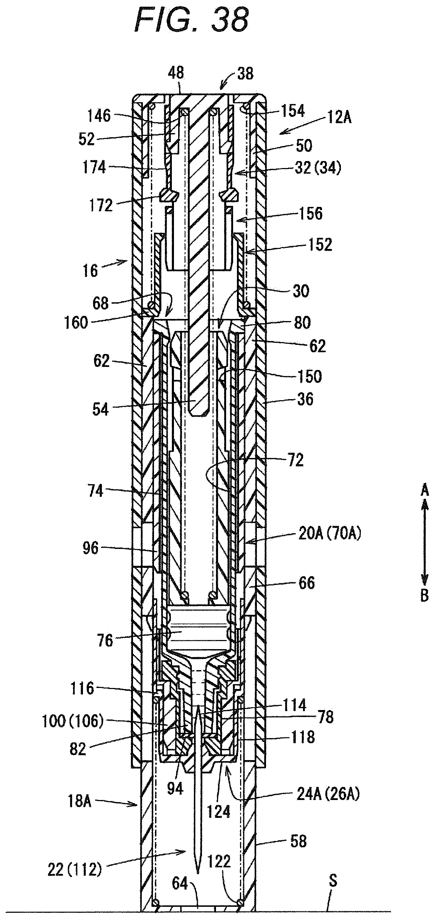

As illustrated in Fig. 38, the extending portions 62 of the needle cover 18A are in contact with the distal end (flange portion 160) of the locking member 152, and thus, the movement of the needle cover 18A in the proximal direction (direction of arrow A) is also inhibited. After the puncture, the movement of the needle cover 18A in the proximal direction is inhibited. For this reason, even when a force in the proximal direction acts on the needle cover 18A, the first needle portion 112 of the double-ended needle 22 is not exposed to the outside from the needle cover 18A, and thus, safety is ensured.

As described above, the liquid medicine administration device 12A of the liquid medicine administration system 10A includes the syringe holder 70A that is movable in the axial direction of the housing 16 and accommodates and holds the syringe 68 therein, and the syringe holder 70A includes the holding portion 106 that holds the distal portion of the syringe 68 coaxially with the distal portion of the syringe holder 70A by elastically biasing the distal portion of the syringe 68 radially inward. Therefore, even when the barrel 74 of the syringe 68 is curved by a thermal load due to autoclave sterilization or the like, the holding portion 106 holds the distal portion of the syringe 68 coaxially with the distal portion of the syringe holder 70A, so that the syringe 68 can be moved in the distal direction together with the syringe holder 70A within the housing 16, and the distal end of the syringe 68 and the second needle portion 114 of the double-ended needle 22 can be reliably connected. Moreover, the clearance formed between the outer peripheral surface of the barrel 74 and the holder body 96 of the syringe holder 70A appropriately absorbs the curvature of the barrel 74, whereby the holding portion 106 can satisfactorily exhibit its function (function of ensuring coaxiality).

The holding portion 106 includes the plurality of flexible arms 108 that presses the outer peripheral surface of the distal portion of the syringe 68 radially inward at a plurality of positions in the circumferential direction. With this configuration, the plurality of flexible arms 108 presses the distal portion of the syringe 68 radially inward, whereby the coaxiality between the distal portion of the syringe holder 70A and the distal portion of the syringe 68 can be effectively enhanced.

The plurality of flexible arms 108 is provided at equal intervals along the circumferential direction of the syringe holder 70A. With this configuration, the distal portion of the syringe 68 can be uniformly pressed toward the axial center by the plurality of flexible arms 108, whereby the coaxiality between the distal portion of the syringe holder 70A and the distal portion of the syringe 68 can be further enhanced.

The syringe 68 has the cap 78 attached to the distal end of the barrel 74, and the plurality of flexible arms 108 presses the outer peripheral surface of the cap 78. With this configuration, the flexible arms 108 press the cap 78 that generally has less thermal deformation than the barrel 74, whereby the coaxiality between the distal portion of the syringe holder 70A and the distal portion of the syringe 68 can be effectively enhanced.

The outer peripheral surface of the cap 78 has the stepped portion 90 between the large-diameter portion 86 located at the proximal end and the small-diameter portion 88 located at the distal end, and the plurality of flexible arms 108 is engaged with the distal side of the stepped portion 90. With this configuration, the distal portion of the syringe holder 70A and the distal portion of the syringe 68 can be positioned in the axial direction by the plurality of flexible arms 108. Therefore, the plurality of flexible arms 108 can be brought into contact with the outer peripheral surface of the cap 78 at appropriate positions. Thus, coaxiality between the distal portion of the syringe holder 70A and the distal portion of the syringe 68 can be effectively enhanced.

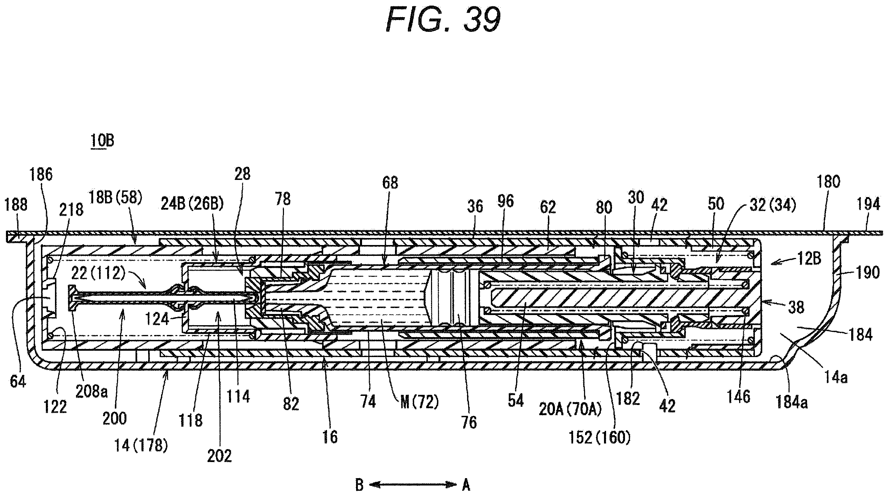

Next, a liquid medicine administration system 10B will be described with reference to Figs. 39 to 44. Note that the same components as those of the liquid medicine administration system 10A are denoted by the same reference numerals, and the detailed description thereof will be omitted.

The liquid medicine administration system 10B illustrated in Fig. 39 includes a liquid medicine administration device 12B and a packaging container 14 that accommodates the liquid medicine administration device 12B and has an internal space 14a that is sterilized.

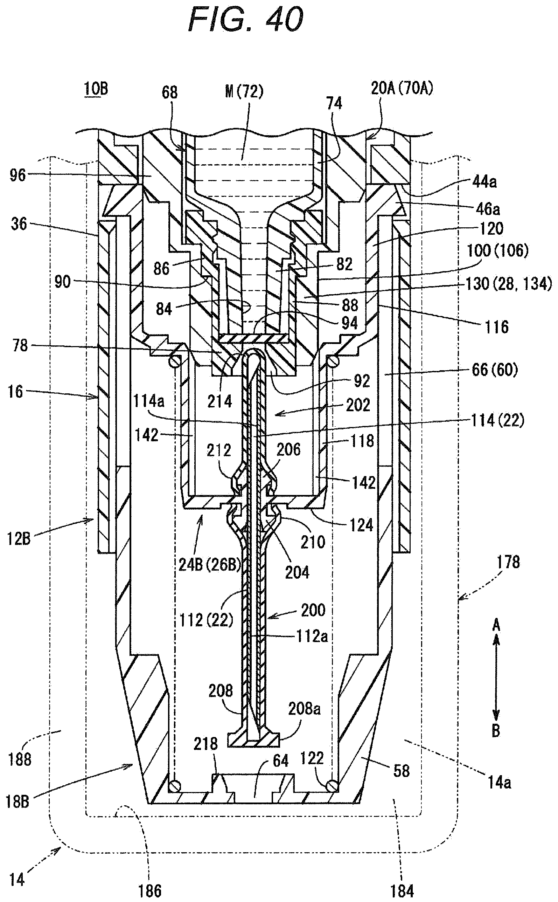

As illustrated in Figs. 39 and 40, the liquid medicine administration device 12B includes a needle unit 26B, and the needle unit 26B includes a needle hub 24B, a first antibacterial cover 200 attached to a first needle portion 112 of a double-ended needle 22 supported by the needle hub 24B, and a second antibacterial cover 202 attached to a second needle portion 114 of the double-ended needle 22. In the double-ended needle 22, the antibacterial cover may be attached only to the first needle portion 112 that punctures the skin S of the user.

As illustrated in Fig. 40, the needle hub 24B includes a first annular protrusion 204 protruding in the distal direction (direction of arrow B) from the center of the support portion 124 and a second annular protrusion 206 protruding in the proximal direction (direction of arrow A) from the center of the support portion 124. The first annular protrusion 204 supports the first needle portion 112, and the second annular protrusion 206 supports the second needle portion 114.

The first and second antibacterial covers 200 and 202 are each formed in a tubular shape from an elastic material such as silicone rubber, butyl rubber, or elastomer, for example, and can be contracted in the axial direction. In a state where the first and second antibacterial covers 200 and 202 are attached to the double-ended needle 22, the double-ended needle 22 is irradiated with electron beams or radial rays together with the needle hub 24B to be sterilized by either electron beam sterilization or radiation sterilization. The first and second antibacterial covers 200 and 202 are formed of a material that can transmit electron beams or radial rays.

The first antibacterial cover 200 includes a first cover distal portion 208 that covers the distal portion of the first needle portion 112 in the initial state illustrated in Fig 40 before the puncture operation is performed, and a first cover proximal portion 210 connected to the support portion 124 of the needle hub 24B. The first cover distal portion 208 has a base 208a orthogonal to the extending direction of the first antibacterial cover 200. The first cover proximal portion 210 is opened, is engaged with the outer periphery of the first annular protrusion 204 of the needle hub 24B, and is connected to and held by the first annular protrusion 204.

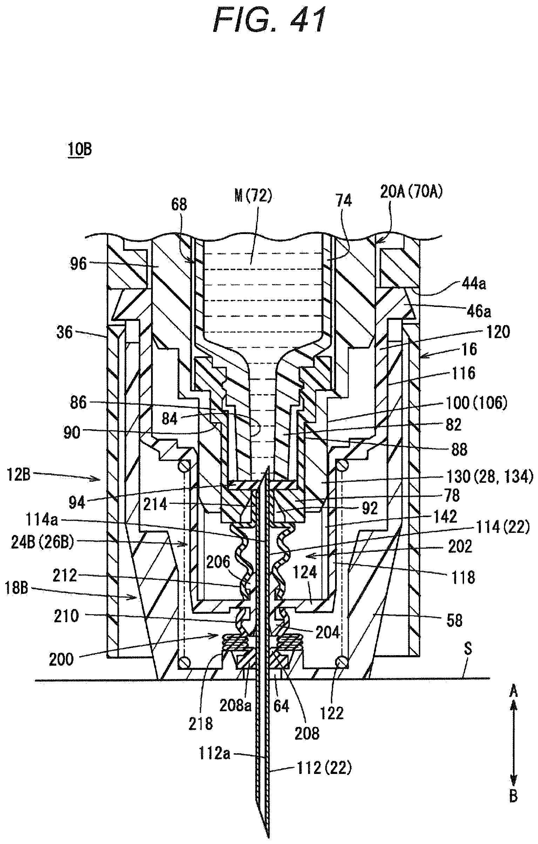

The base 208a is formed in a disk shape, and when the first antibacterial cover 200 is folded in the axial direction at the time of puncture with the first needle portion 112 illustrated in Fig. 41, the base 208a is accommodated and held in a receiving portion 218 formed on the inner surface of the distal end of a main body 58 of the needle cover 18B. The receiving portion 218 is formed at the center of the inner surface of the distal end of the needle cover 18B, and has a cup shape annularly upright in the proximal direction. A distal end opening 64 is opened at the center of the receiving portion 218.

The second antibacterial cover 202 includes a second cover distal portion 212 connected to the support portion 124 of the needle hub 24B, and a second cover proximal portion 214 that covers the proximal portion (needle tip) of the second needle portion 114 in the initial state illustrated in Fig 40 before the puncture operation is performed.

The second cover distal portion 212 is opened, is engaged with the outer periphery of the second annular protrusion 206 of the needle hub 24B, and is connected to and held by the second annular protrusion 206. In the initial state, the second cover proximal portion 214 is inserted into an insertion hole 92 of a cap 78.

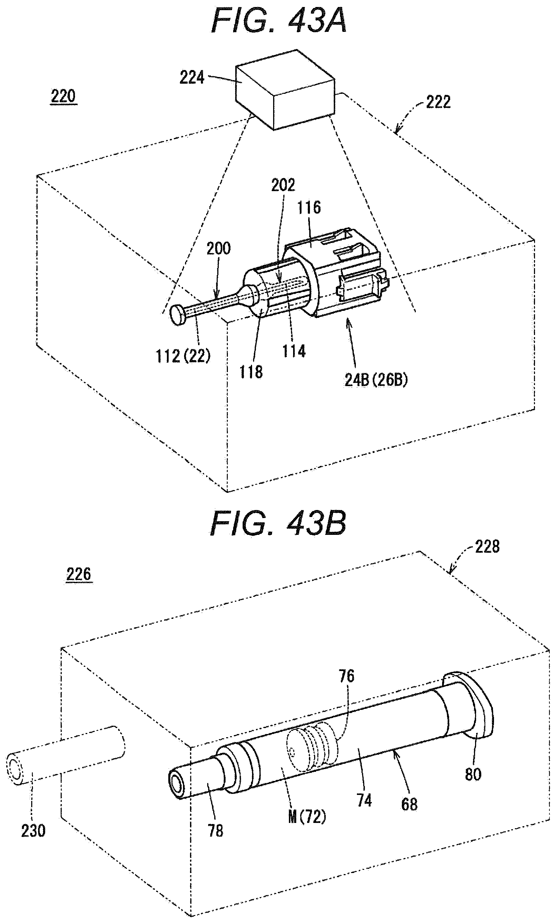

Next, a case where a sterilization treatment is performed at the time of manufacturing the liquid medicine administration system 10B including the above-described liquid medicine administration device 12B will be described with reference to Figs. 43A to 44.

First, before the liquid medicine administration device 12B is assembled, electron beam sterilization or radiation sterilization that is a first sterilization method is performed on the needle unit 26B including the double-ended needle 22 (first sterilization process) as illustrated in Fig. 43A.

As illustrated in Fig. 43A, the needle unit 26B including the double-ended needle 22 in which the first antibacterial cover 200 is attached to the first needle portion 112 and the second antibacterial cover 202 is attached to the second needle portion 114 is inserted into a first sterilization chamber 222 of a first sterilization device 220. In the first sterilization chamber 222, electron beams or radial rays are emitted from an irradiation source 224 to the needle unit 26B for a predetermined time.

Electron beams or radial rays pass through the first and second antibacterial covers 200 and 202, whereby the first and second needle portions 112 and 114 are sterilized by electron beams or radial rays. As a result, the sterility of the double-ended needle 22 covered with the first and second antibacterial covers 200 and 202 is ensured.

Next, before the liquid medicine administration device 12B is assembled, autoclave sterilization (high-pressure steam sterilization), which is a second sterilization method, is performed on the syringe 68 (second sterilization process).

As illustrated in Fig. 43B, the syringe 68 containing a liquid medicine M that is filled within a medicine chamber 72 of a barrel 74 is put into a second sterilization chamber 228 of a second sterilization device 226. High pressure water vapor is supplied into the second sterilization chamber 228 through a pipe 230.

The syringe 68 is heated to a predetermined temperature in the second sterilization chamber 228 by high-pressure water vapor, by which the syringe 68 is sterilized.

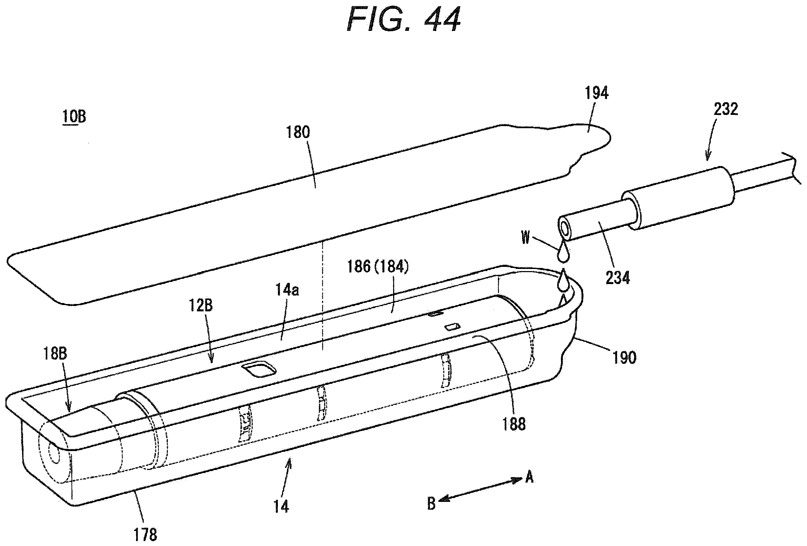

Finally, after assembling the liquid medicine administration device 12B including the needle unit 26B sterilized with the first sterilization method and the syringe 68 sterilized with the second sterilization method, the liquid medicine administration device 12B is accommodated in an accommodation recess 184 of the packaging container 14 as illustrated in Fig. 44, and the internal space 14a of the packaging container 14 is sterilized with a third sterilization method (third sterilization process).

Specifically, in a state where the liquid medicine administration device 12B illustrated in Fig. 44 is accommodated in the accommodation recess 184 of the packaging container 14, sterile water W is dropped into the accommodation recess 184 (internal space 14a) from a supply nozzle 234 of a dropping device 232 through the opening 186 of the container body 178. The sterile water W is hydrogen peroxide water. A predetermined amount of sterile water W is dropped into the accommodation recess 184 from the supply nozzle 234, and then the sealing film 180 is fused to the opening edge 188 of the container body 178, whereby the container body 178 is sealed.

The liquid medicine administration system 10B is left in an environment with room temperature or higher, by which the sterile water W is gradually vaporized in the packaging container 14 and filled in the internal space 14a of the packaging container 14 as a sterile gas. The filled sterile gas can appropriately sterilize the inside and the outer surface of the liquid medicine administration device 12B. As a result, the lid body 94 provided at the distal end of the medicine chamber 72 in the syringe 68 and the second needle portion 114 of the double-ended needle 22 that is to puncture the lid body 94 are sterilized, and sterility between the lid body 94 and the second needle portion 114 can be ensured.

Note that the needle body of the liquid medicine administration device 12B to which the antibacterial cover is attached is not limited to the double-ended needle 22 having the first and second needle portions 112 and 114. The needle body may be a single-ended needle connected in advance to the distal end of the syringe 68.

Next, the operation of the sterilized liquid medicine administration device 12B will be described. Note that the detailed description of the same operation as that of the liquid medicine administration device 12A is omitted.

When the user presses the distal end of the needle cover 18B against the skin S and moves the needle cover 18B in the proximal direction relative to the housing 16 from the initial state before the puncture operation illustrated in Fig. 40, the base 208a of the first antibacterial cover 200 is inserted into the receiving portion 218 of the needle cover 18B along with the movement of the needle cover 18B in the proximal direction, and then the first cover distal portion 208 is pushed up in the proximal direction (direction of arrow A) together with the needle cover 18B via the base 208a.

As illustrated in Fig. 41, as the first cover distal portion 208 including the base 208a moves in the proximal direction, the needle tip of the first needle portion 112 punctures the base 208a, penetrates the base 208a, and protrudes in the distal direction. As the needle cover 18B moves in the proximal direction, the first antibacterial cover 200 relatively moves from the first cover distal portion 208 toward the first cover proximal portion 210, and the first cover distal portion 208 is folded and contracted in the axial direction toward the first cover proximal portion 210. The first antibacterial cover 200 comes into contact with the receiving portion 218 of the needle cover 18B and is folded in the proximal direction so as to be stacked in the axial direction.

When the puncture of the skin S with the first needle portion 112 is completed, the entire portion between the base 208a of the first antibacterial cover 200 and the first cover proximal portion 210 is folded in the axial direction.

When the drive unit 34 is activated and the syringe unit 20A moves in the distal direction together with the plunger 30, the lid body 94 comes into contact with the second cover proximal portion 214 of the second antibacterial cover 202 along with the movement of the syringe unit 20A, and then, the syringe unit 20A further moves in the distal direction, so that the needle tip of the second needle portion 114 punctures and penetrates the second cover proximal portion 214. The needle tip of the second needle portion 114 punctures the lid body 94, whereby the distal portion of the syringe 68 and the second needle portion 114 are connected.

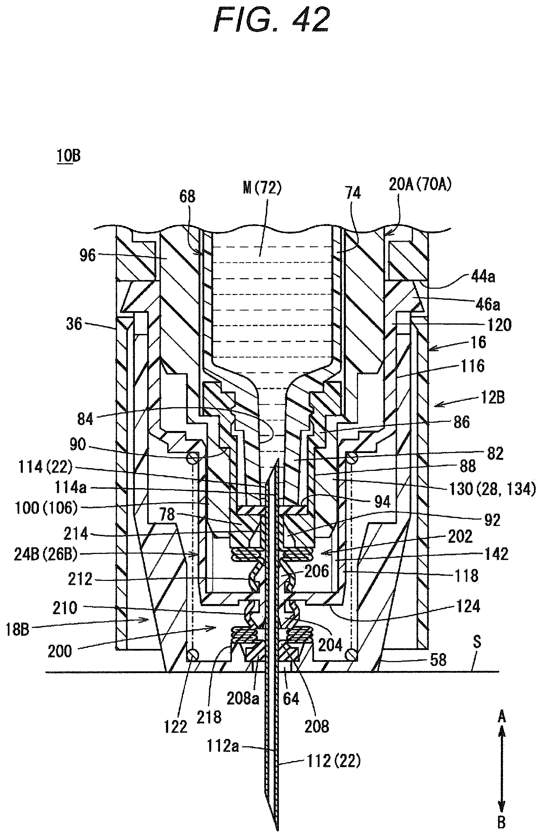

As illustrated in Fig. 41, after the second needle portion 114 and the syringe 68 are connected, the syringe unit 20A further moves in the distal direction. Thus, the second cover proximal portion 214 is pushed in the distal direction by the end face of the lid body 94, and the second cover proximal portion 214 of the second antibacterial cover 202 relatively moves toward the second cover distal portion 212 and is folded and contracted in the axial direction toward the second cover distal portion 212 (see Fig. 42).

The second antibacterial cover 202 comes into contact with the proximal end of the second annular protrusion 206 and is folded in the distal direction so as to be stacked in the axial direction.

When the distal end of the syringe unit 20A comes into contact with the support portion 124 of the needle hub 24B and the connection between the double-ended needle 22 and the syringe 68 is completed, the portion between the second cover distal portion 212 and the second cover proximal portion 214 of the second antibacterial cover 202 is folded in the axial direction.

As described above, in the liquid medicine administration system 10B, the first needle portion 112 and the second needle portion 114 of the double-ended needle 22 are covered with the first antibacterial cover 200 and the second antibacterial cover 202, respectively, whereby the double-ended needle 22 can be sterilized before the liquid medicine administration device 12B is assembled. On the other hand, the syringe 68 can be sterilized by a sterilization method different from the sterilization of the double-ended needle 22 before assembly of the liquid medicine administration device 12B. Therefore, it is possible to select a suitable sterilization method for each of the double-ended needle 22 and the syringe 68, and thus, it is possible to avoid a mismatch of the sterilization method for the double-ended needle 22 and the syringe 68.

Therefore, a decrease in function of the syringe 68 and the double-ended needle 22 due to the sterilization treatment can be prevented. The inside of the packaging container 14 containing the liquid medicine administration device 12B is sterilized with the third sterilization method using the sterile water W, whereby sterility between the lid body 94 of the syringe 68 and the double-ended needle 22 can also be ensured.

The liquid medicine administration system 10B can be sterilized without using a large-scale sterile environment facility that requires a corresponding installation and maintenance cost, and thus, it is possible to ensure the sterility of the entire liquid medicine administration device 12B at low cost until immediately before the user uses the liquid medicine administration device 12B.

The double-ended needle 22 (needle unit 26B) to which the first and second antibacterial covers 200 and 202 are attached is subjected to electron beam sterilization or radiation sterilization, by which it is possible to avoid thermal influence on the support portion 124 of the needle hub 24B supporting the double-ended needle 22 by adhesion or the like.

The syringe 68 containing the liquid medicine M filled in the medicine chamber 72 is subject to autoclave sterilization with high-pressure water vapor, by which it is possible to avoid denaturation of the liquid medicine M.

The sterile water W is dropped into the container body 178 of the packaging container 14 and is vaporized in the internal space 14a of the container body 178, whereby the inside of the container body 178 can be entirely brought into a sterile atmosphere. Therefore, the sterility of the entire liquid medicine administration device 12B can be ensured until the sealing film 180 is peeled off from the container body 178 to open the packaging container 14.

Hydrogen peroxide water is used as the sterile water W. Therefore, when the liquid medicine administration system 10B is left in an environment with room temperature or higher, the hydrogen peroxide water can be vaporized in the internal space 14a of the packaging container 14 and filled in the packaging container 14. The filled hydrogen peroxide gas can appropriately sterilize the inside and the outer surface of the liquid medicine administration device 12B.

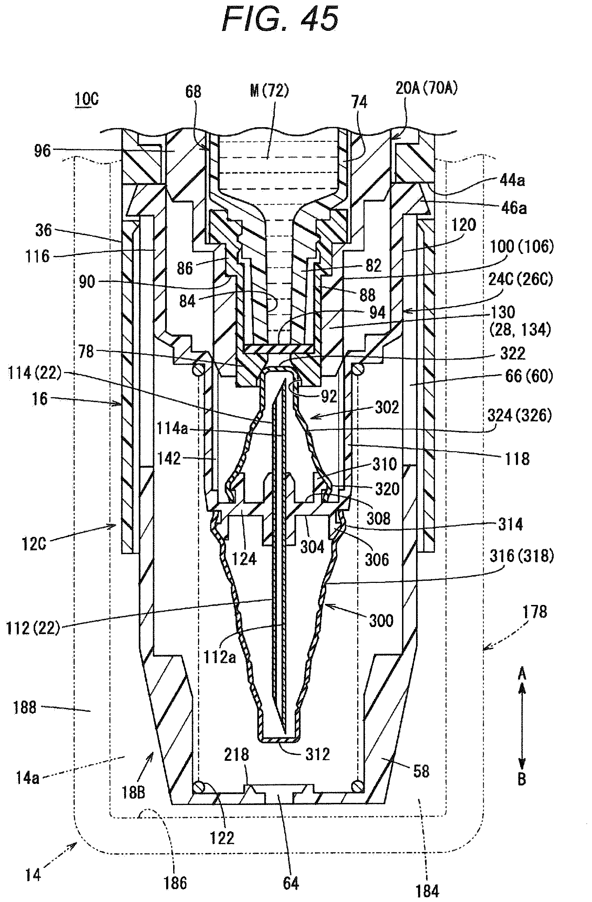

Next, a liquid medicine administration system 10C will be described with reference to Figs. 45 to 48. Note that the same components as those of the liquid medicine administration systems 10A and 10B are denoted by the same reference numerals, and the detailed description thereof will be omitted.

The liquid medicine administration system 10C illustrated in Fig. 45 includes a liquid medicine administration device 12C and a packaging container 14 that accommodates the liquid medicine administration device 12C and has an internal space 14a that is sterilized.

A needle unit 26C of the liquid medicine administration device 12C includes a needle hub 24C, a first antibacterial cover 300 attached to a first needle portion 112 of a double-ended needle 22 supported by the needle hub 24C, and a second antibacterial cover 302 attached to a second needle portion 114 of the double-ended needle 22. The first and second antibacterial covers 300 and 302 are each formed in a tubular shape from an elastic material such as silicone rubber, butyl rubber, or elastomer. In the double-ended needle 22, the antibacterial cover may be attached only to the first needle portion 112 that punctures the skin S of the user.

The needle hub 24C includes a first accommodation portion 304 and a first annular protrusion 306 disposed on the distal side of the support portion 124, and a second accommodation portion 308 and a second annular protrusion 310 disposed on the proximal side of the support portion 124.

The first accommodation portion 304 is a recess capable of accommodating a first bellows portion 316 of the first antibacterial cover 300 when the first bellows portion 316 is folded. The first accommodation portion 304 opens in the distal direction (direction of arrow B).

The first annular protrusion 306 surrounds the first needle portion 112 and protrudes from the support portion 124 in the distal direction (direction of arrow B), and a first cover proximal portion 314 of the first antibacterial cover 300 described later is held on the outer periphery thereof. The first accommodation portion 304 is disposed inside the first annular protrusion 306.

The second accommodation portion 308 is a recess capable of accommodating a second bellows portion 324 of the second antibacterial cover 302 when the second bellows portion 324 is folded. The second accommodation portion 308 opens in the proximal direction (direction of arrow A).

The second annular protrusion 310 surrounds the second needle portion 114 and protrudes from the support portion 124 in the proximal direction (direction of arrow A), and a second cover distal portion 320 of the second antibacterial cover 302 described later is held on the outer periphery thereof. The second accommodation portion 308 is disposed inside the second annular protrusion 310.

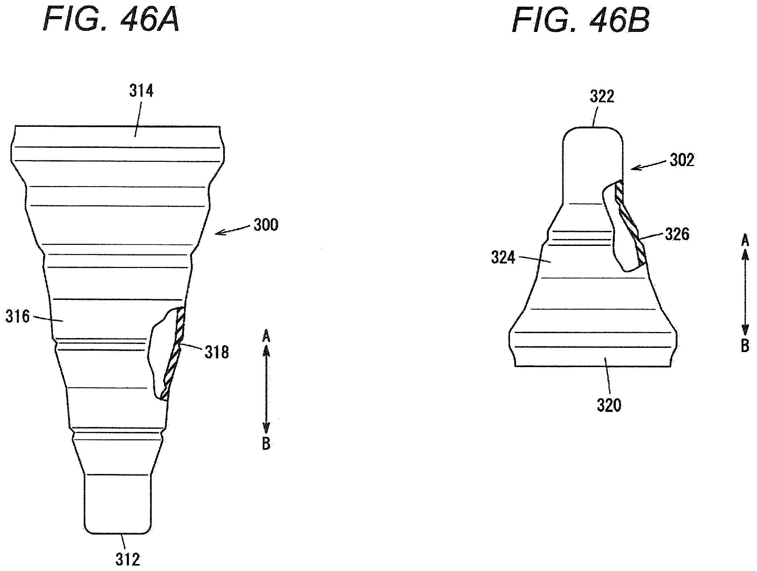

The first antibacterial cover 300 includes the first cover distal portion 312 covering the distal portion of the first needle portion 112, the first cover proximal portion 314 connected to the first annular protrusion 306 of the needle hub 24C, and the first bellows portion 316 provided between the first cover distal portion 312 and the first cover proximal portion 314 in an initial state illustrated in Figs. 45 and 46A before the puncture operation is performed.

The first cover distal portion 312 has a smaller diameter than the first cover proximal portion 314. The first cover proximal portion 314 is opened, is engaged with the outer periphery of the first annular protrusion 306 of the needle hub 24C, and is held by the first annular protrusion 306.

As illustrated in Fig. 46A, the first bellows portion 316 is formed in a truncated cone shape, and has a plurality of first folds 318 provided along the axial direction of the first antibacterial cover 300 and recessed in the radial direction orthogonal to the axial direction. The first bellows portion 316 is foldable in the radial direction via the plurality of first folds 318.

The second antibacterial cover 302 includes the second cover distal portion 320 connected to the second annular protrusion 310 of the needle hub 24C, the second cover proximal portion 322 covering the proximal portion (needle tip) of the second needle portion 114 in an initial state illustrated in Figs. 45 and 46B before the puncture operation is performed, and a second bellows portion 324 provided between the second cover distal portion 320 and the second cover proximal portion 322.

The second cover distal portion 320 is opened, is engaged with the outer periphery of the second annular protrusion 310 of the needle hub 24C, and is held by the second annular protrusion 310. The second cover proximal portion 322 has a smaller diameter than the second cover distal portion 320. In an initial state before puncturing is performed, the second cover proximal portion 322 is inserted into and held in an insertion hole 92 of a cap 78.

As illustrated in Fig. 46B, the second bellows portion 324 is formed in a truncated cone shape, and has a plurality of second folds 326 provided along the axial direction of the second antibacterial cover 302 and recessed in the radial direction orthogonal to the axial direction. The second bellows portion 324 is foldable in the radial direction via the plurality of second folds 326.

Next, the operation of the liquid medicine administration device 12C of the liquid medicine administration system 10C will be described. Note that the detailed description of the same operation as that of the liquid medicine administration devices 12A and 12B is omitted.

When the user presses the distal end of the needle cover 18B against the skin S and moves the needle cover 18B in the proximal direction relative to a housing 16 from the initial state illustrated in Fig. 45 before the puncture operation, the first cover distal portion 312 of the first antibacterial cover 300 comes in contact with the receiving portion 218 of the needle cover 18B along with the movement of the needle cover 18B in the proximal direction, and then the first cover distal portion 312 is pushed up in the proximal direction (direction of arrow A) together with the needle cover 18B.

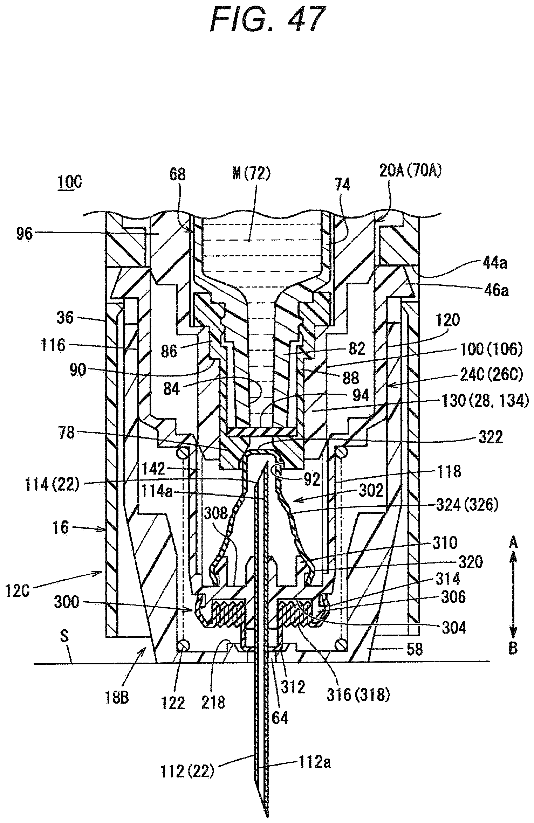

With the movement of the needle cover 18B in the proximal direction, the first cover distal portion 312 relatively moves toward the first cover proximal portion 314, and the first bellows portion 316 is folded in the radial direction starting from the plurality of first folds 318 as illustrated in Fig. 47. At this time, due to the first bellows portion 316 having a truncated cone shape, the first antibacterial cover 300 can be folded smoothly in such a manner that the first cover distal portion 312 is located inside the first cover proximal portion 314 in the radial direction.

When the needle tip of the first needle portion 112 punctures the first cover distal portion 312, the first needle portion 112 protrudes to the outside of the first cover distal portion 312 and protrudes from the distal end of the needle cover 18B through the distal end opening 64 to puncture the skin S.

As illustrated in Fig. 47, as the needle cover 18B further moves in the proximal direction, the first bellows portion 316 is sequentially folded radially inward on the radially inner side of the first cover proximal portion 314, and the folded first bellows portion 316 is accommodated in the first accommodation portion 304 so as to be stacked in the radial direction. The first antibacterial cover 300 contracts in the axial direction such that the first cover distal portion 312 approaches the first cover proximal portion 314 as the first bellows portion 316 is folded in the radial direction.

When the puncture by the first needle portion 112 is completed, the entire first bellows portion 316 of the first antibacterial cover 300 is folded in the radial direction and accommodated in the first accommodation portion 304.

When a drive unit 34 is activated and a syringe unit 20A moves in the distal direction together with a plunger 30, the insertion hole 92 of the cap 78 comes into contact with the second cover proximal portion 322 of the second antibacterial cover 302 along with the movement of the syringe unit 20A in the distal direction, and then, the syringe unit 20A further moves in the distal direction, so that the needle tip of the second needle portion 114 punctures and penetrates the second cover proximal portion 322. The needle tip of the second needle portion 114 punctures the lid body 94, whereby the distal portion of the syringe 68 and the second needle portion 114 are connected.

After the second needle portion 114 and the syringe 68 are connected, the syringe unit 20A further moves in the distal direction. Thus, the second cover proximal portion 322 is pushed in the distal direction by the cap 78, and the second cover proximal portion 322 relatively moves toward the second cover distal portion 320, and accordingly, the second bellows portion 324 of the second antibacterial cover 302 is folded in the racial direction starting from the second folds 326. At this time, due to the second bellows portion 324 having a truncated cone shape, the second antibacterial cover 302 can be folded smoothly in such a manner that the second cover proximal portion 322 is located inside the second cover distal portion 320 in the radial direction.

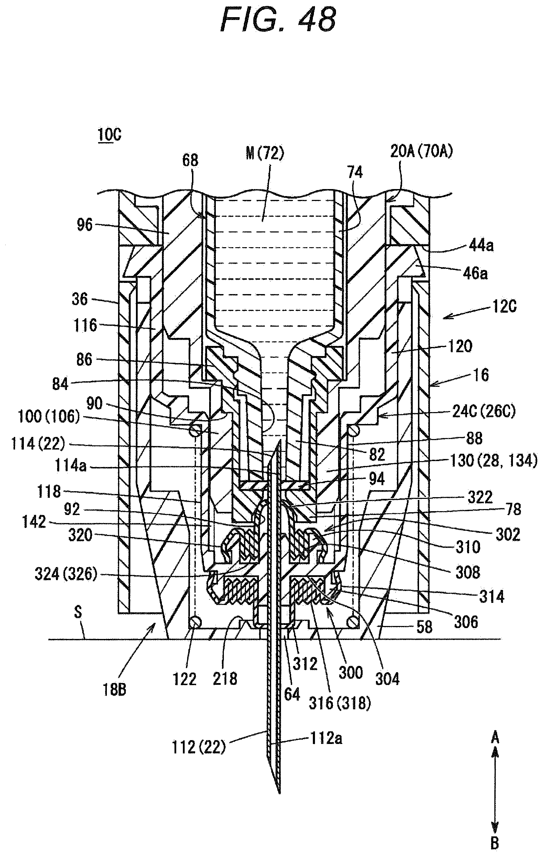

As the syringe unit 20A further moves in the distal direction, the second bellows portion 324 is sequentially folded radially inward on the radially inner side of the second cover distal portion 320, and the folded second bellows portion 324 is accommodated in the second accommodation portion 308 so as to be stacked in the radial direction as illustrated in Fig. 48. The second antibacterial cover 302 contracts in the axial direction such that the second cover proximal portion 322 approaches the second cover distal portion 320 as the second bellows portion 324 is folded in the radial direction.

When the connection between the second needle portion 114 of the double-ended needle 22 and the syringe 68 is completed, the entire second bellows portion 324 of the second antibacterial cover 302 is folded in the radial direction and accommodated in the second accommodation portion 308.

The liquid medicine administration system 10C described above may be sterilized by the first to third sterilization methods performed on the liquid medicine administration system 10B.

Note that the needle body of the liquid medicine administration device 12C to which the antibacterial cover is attached is not limited to the double-ended needle 22 having the first and second needle portions 112 and 114. The needle body may be a single-ended needle connected in advance to the distal end of the syringe 68.

The first antibacterial cover 300 may be formed such that the first cover proximal portion 314 is smaller in diameter than the first cover distal portion 312. The second antibacterial cover 302 may be formed such that the second cover distal portion 320 is smaller in diameter than the second cover proximal portion 322. In this case, when the first and second antibacterial covers 300 and 302 are folded in the axial direction, the first and second bellows portions 316 and 324 can also be folded in the radial direction. In this case, the first cover distal portion 312 is accommodated on the radially outer side of the first cover proximal portion 314, and the second cover proximal portion 322 is accommodated on the radially outer side of the second cover distal portion 320.

As described above, the liquid medicine administration device 12C of the liquid medicine administration system 10C includes the first and second antibacterial covers 300 and 302 attached to the first and second needle portions 112 and 114 of the double-ended needle 22. The first antibacterial cover 300 includes the first cover proximal portion 314 connected to the needle hub 24C, the first cover distal portion 312 having a diameter different from that of the first cover proximal portion 314 and covering the distal portion of the first needle portion 112 in the initial state, and the first bellows portion 316 disposed between the first cover distal portion 312 and the first cover proximal portion 314. The second antibacterial cover 302 includes the second cover distal portion 320 connected to the needle hub 24C, the second cover proximal portion 322 having a diameter different from that of the second cover proximal portion 322 and covering the distal portion of the second needle portion 114 in the initial state, and the second bellows portion 324 disposed between the second cover distal portion 320 and the second cover proximal portion 322.

When the skin S of the user is punctured with the second needle portion 114 of the double-ended needle 22, the first cover distal portion 312 and the first cover proximal portion 314 of the first antibacterial cover 300 relatively move in the axial direction, whereby the first bellows portion 316 is folded in the radial direction. Therefore, the first and second antibacterial covers 300 and 302 foldable in the radial direction can have a shorter axial dimension when folded as compared with a conventional antibacterial cover folded in the axial direction.

As a result, both the first antibacterial cover 300 and the second antibacterial cover 302 covering the double-ended needle 22 can be folded in the radial direction, and thus, the axial dimension of the liquid medicine administration device 12C having the double-ended needle 22 can be effectively reduced.

The first bellows portion 316 of the first antibacterial cover 300 and the second bellows portion 324 of the second antibacterial cover 302 are formed into a truncated conical shape. With this configuration, the first and second antibacterial covers 300 and 302 can be folded smoothly, whereby an increase in puncture resistance at the time of puncture due to providing the first and second antibacterial covers 300 and 302 on the double-ended needle 22 is suppressed.

When being folded, the first and second bellows portions 316 and 324 can be respectively accommodated in the first and second accommodation portions 304 and 308 provided inside the first cover proximal portion 314 and the second cover distal portion 320. Therefore, the thickness in the axial direction of the needle hub 24C in the vicinity of the support portion 124 where the first and second bellows portions 316 and 324 are folded can be decreased. Therefore, the axial dimension of the liquid medicine administration device 12C along the axial direction including the needle hub 24C can be reduced.

In the first antibacterial cover 300, the first cover distal portion 312 has a smaller diameter than the first cover proximal portion 314. With this configuration, the first cover distal portion 312 can be located inside of the first cover proximal portion 314 in the radial direction when the first antibacterial cover 300 is folded. Therefore, as compared with a case where the first cover proximal portion 314 has a smaller diameter than the first cover distal portion 312, the outer diameter of the first antibacterial cover 300 can be reduced, which can contribute to a reduction in size of the liquid medicine administration device 12C.

In the second antibacterial cover 302, the second cover proximal portion 322 has a smaller diameter than the second cover distal portion 320. With this configuration, the second cover proximal portion 322 can be located inside of the second cover distal portion 320 in the radial direction when the second antibacterial cover 302 is folded. Therefore, as compared with a case where the second cover distal portion 320 has a smaller diameter than the second cover proximal portion 322, the outer diameter of the second antibacterial cover 302 can be reduced, which can contribute to a reduction in size of the liquid medicine administration device 12C.

Since the plurality of first folds 318 recessed in the radial direction is formed in the first bellows portion 316, the first antibacterial cover 300 can be folded in the axial direction such that the first cover distal portion 312 and the first cover proximal portion 314 are located in the radial direction with the plurality of first folds 318 as starting points. Since the plurality of second folds 326 recessed in the radial direction is formed in the second bellows portion 324, the second antibacterial cover 302 can be folded in the axial direction such that the second cover distal portion 320 and the second cover proximal portion 322 are located in the radial direction with the plurality of second folds 326 as starting points.

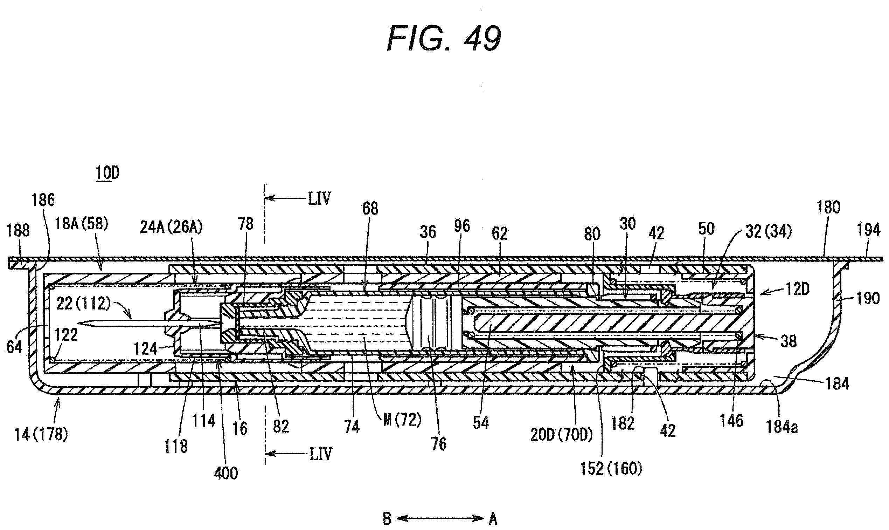

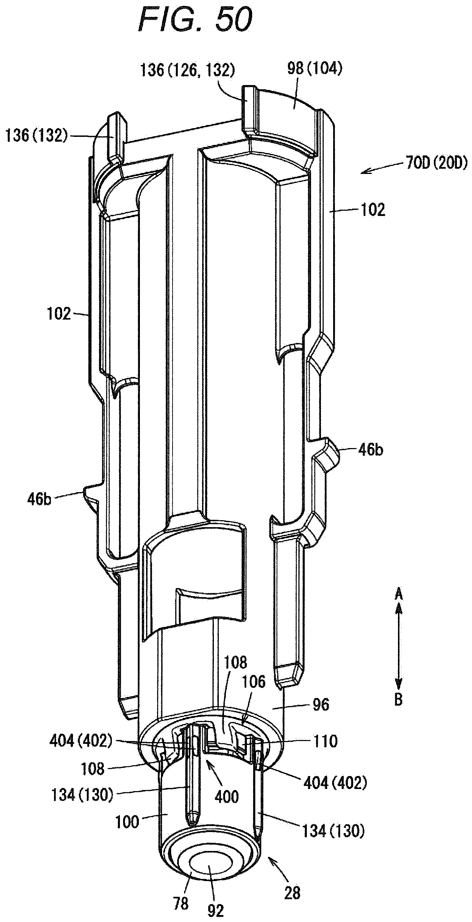

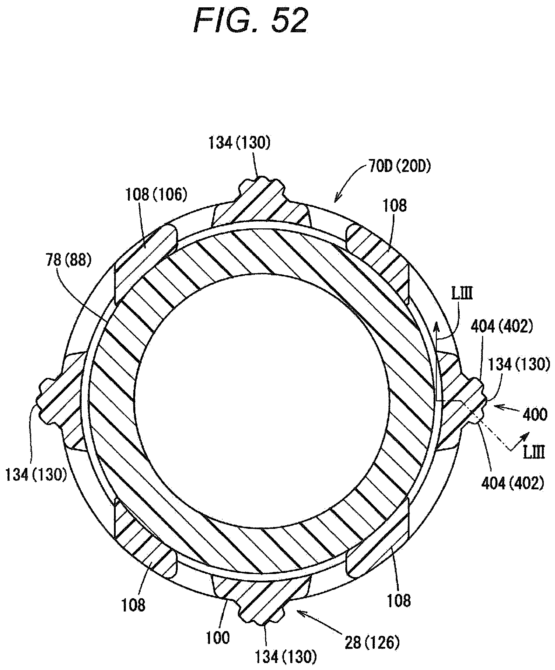

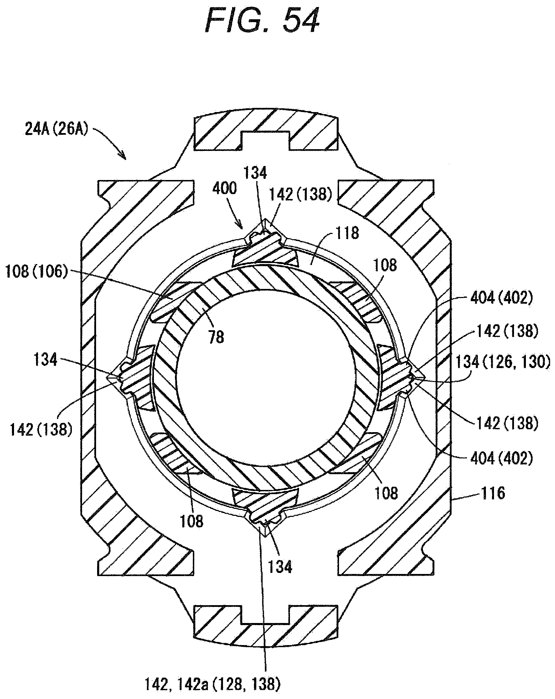

Next, a liquid medicine administration system 10D will be described with reference to Figs. 49 to 55C. Note that the same components as those of the liquid medicine administration systems 10A, 10B, and 10C are denoted by the same reference numerals, and the detailed description thereof will be omitted.

The liquid medicine administration system 10D illustrated in Fig. 49 includes a liquid medicine administration device 12D and a packaging container 14 that accommodates the liquid medicine administration device 12D.

The liquid medicine administration device 12D includes a deceleration mechanism 400 that reduces the moving speed of a syringe unit 20D in the distal direction during the puncture operation. After a second needle portion 114 of a double-ended needle 22 starts to puncture a lid body 94 of the syringe unit 20D and before the connection between the distal end of the syringe unit 20D and the second needle portion 114 is completed, the deceleration mechanism 400 reduces the moving speed of the syringe unit 20D in the distal direction.

As illustrated in Figs. 49 to 51, the deceleration mechanism 400 includes a deceleration portion 402 disposed on the outer peripheral surface of a holder distal cylindrical portion 100 of a syringe holder 70D, and a plurality of the deceleration portions 402 is provided along the circumferential direction of the holder distal cylindrical portion 100.

Each of the deceleration portions 402 is an inclined protrusion 404 disposed at the proximal portion of the holder distal cylindrical portion 100 and provided at the proximal portion of a first guide rib 134.

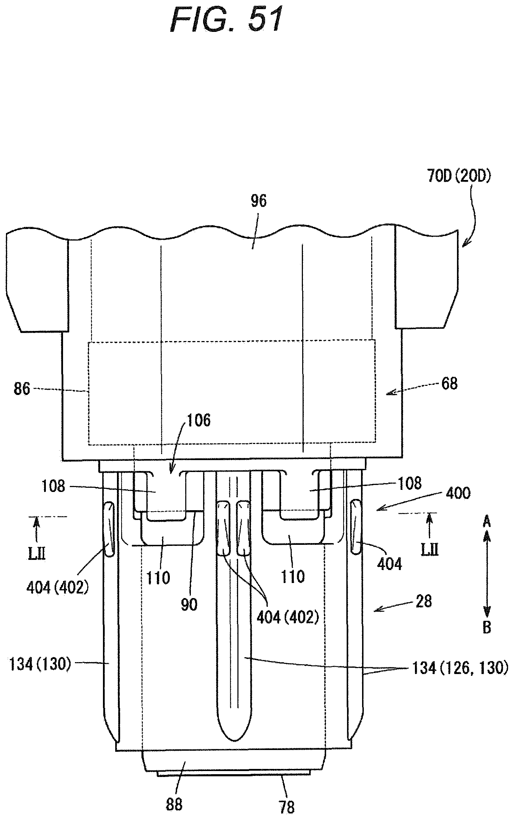

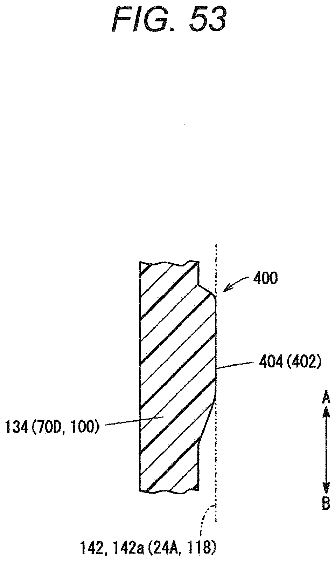

As illustrated in Figs. 51 to 53, the inclined protrusion 404 protrudes outward from the first guide rib 134 in a direction orthogonal to the first guide rib 134, and is inclined so that the protruding height from the first guide rib 134 increases in the proximal direction (direction of arrow A) (see Fig. 53). When the syringe unit 20D moves in the distal direction and the first guide ribs 134 are inserted into first guide grooves 142, each of the inclined protrusions 404 is fitted to an inner surface 142a of the corresponding first guide groove 142, and thus, the insertion length of the first guide rib 134 with respect to the first guide groove 142 increases. The inclined protrusions 404 gradually increase the sliding resistance between the syringe holder 70D and a needle hub 24A.

The inclined protrusion 404 of the deceleration portion 402 may be provided on the inner surface 142a of the first guide groove 142 into which the first guide rib 134 is inserted instead of the first guide rib 134. In this case, the inclined protrusion 404 is disposed at the distal portion of the first guide groove 142. That is, it is sufficient that the deceleration portion 402 is provided in at least one of the proximal portion of the outer peripheral surface of the holder distal cylindrical portion 100 of the syringe holder 70D and the distal portion of the inner peripheral surface of the guide cylindrical portion 118 of the needle hub 24A.

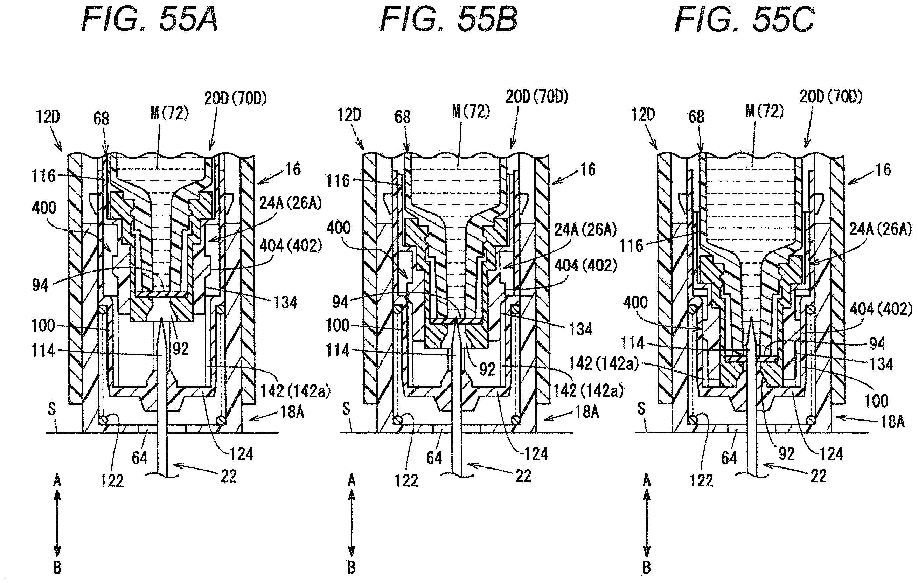

During the puncture operation by the liquid medicine administration device 12D, the syringe unit 20D moves in the distal direction together with a plunger 30 by the resilient force of an injection spring 146, and the distal portions of the first guide ribs 134 of the syringe holder 70D start to be inserted into the first guide grooves 142 from the proximal portions of the first guide grooves 142 of the needle hub 24A as illustrated in Fig. 55A. In this case, the inclined protrusions 404 are not yet inserted into the first guide grooves 142.

As illustrated in Fig. 55B, after the syringe unit 20D moves in the distal direction and the second needle portion 114 of the double-ended needle 22 starts to puncture the distal end (lid body 94) of a syringe 68, the inclined protrusions 404 start to contact the inner surfaces 142a of the first guide grooves 142. Thus, the sliding resistance between the inclined protrusions 404 and the first guide grooves 142 gradually increases.

Therefore, before the connection between the distal end of the syringe unit 20D and the second needle portion 114 is completed, the deceleration portions 402 gradually reduce the moving speed of the syringe unit 20D in the distal direction. The syringe unit 20D that reduces in moving speed further moves in the distal direction, and the distal end of the syringe unit 20D comes into contact with a support portion 124 of the needle hub 24A serving as a stopper as illustrated in Fig. 55C. Thus, the syringe unit 20D stops, and the connection between the syringe unit 20D and the second needle portion 114 is completed.

That is, the deceleration mechanism 400 reduces the moving speed of the syringe unit 20D in the distal direction from when the second needle portion 114 starts to puncture the distal end of the syringe 68 as illustrated in Fig. 55B to when the connection between the second needle portion 114 and the distal end of the syringe 68 is completed as illustrated in Fig. 55C.

The deceleration portion 402 may be directly disposed on the outer peripheral surface of the holder distal cylindrical portion 100 provided with the first guide rib 134, or may be directly disposed on the inner peripheral surface of the guide cylindrical portion 118 provided with the first guide groove 142. That is, it is sufficient that the deceleration portion 402 is provided in at least one of the proximal portion of the outer peripheral surface of the holder distal cylindrical portion 100 and the distal portion of the inner peripheral surface of the guide cylindrical portion 118.

As described above, when the syringe unit 20D moves in the distal direction during the puncture operation by the liquid medicine administration device 12D, the deceleration mechanism 400 reduces the moving speed of the syringe unit 20D, whereby it is possible to decrease an impact received by the syringe unit 20D when the syringe unit 20D stops with the distal end thereof coming into contact with the needle hub 24A. As a result, it is possible to prevent the syringe unit 20D from being damaged due to the impact.

The deceleration mechanism 400 can appropriately reduce the moving speed of the syringe unit 20D with a simple configuration by increasing the sliding resistance of the syringe unit 20D during the movement of the syringe unit 20D in the distal direction.

The deceleration mechanism 400 includes the deceleration portion 402 provided on the outer peripheral surface of the holder distal cylindrical portion 100 of the syringe holder 70D. The deceleration portion 402 protrudes from the outer peripheral surface of the holder distal cylindrical portion 100 and is formed so as to be able to contact the inner peripheral surface of the guide cylindrical portion 118 of the needle hub 24A into which the holder distal cylindrical portion 100 is inserted. With this configuration, when the syringe holder 70D moves in the distal direction and the holder distal cylindrical portion 100 is inserted into the guide cylindrical portion 118 of the needle hub 24A, the syringe holder 70D and the needle hub 24A come into contact with each other via the deceleration portion 402, whereby the sliding resistance between the syringe holder 70D and the needle hub 24A can be increased. As a result, the moving speed of the syringe unit 20D in the distal direction can be effectively reduced.

Since the deceleration portion 402 is provided on the holder distal cylindrical portion 100 provided on the distal end of the syringe holder 70D, the moving speed of the syringe unit 20D including the syringe holder 70D can be reduced before connection between the distal end of the syringe 68 and the second needle portion 114 of the double-ended needle 22 is completed. Therefore, it is possible to minimize an increase in the time required for connecting the syringe unit 20D and the double-ended needle 22 caused by the arrangement of the deceleration portion 402.

The clearance between the first guide rib 134 and the first guide groove 142 is smaller than the clearance between other members including the syringe holder 70D and the needle hub 24A. Therefore, due to the deceleration portion 402 being disposed on the first guide rib 134, the syringe holder 70D and the needle hub 24A can be more reliably brought into contact with each other to reduce the moving speed via the deceleration portion 402.

The deceleration portion 402 is provided as the inclined protrusion 404 provided on the first guide rib 134. With this configuration, as the syringe unit 20D moves in the distal direction, the inclined protrusion 404 is gradually brought into sliding contact with the inner surface 142a of the first guide groove 142 of the needle hub 24A, and the sliding resistance between the syringe holder 70D and the needle hub 24A is gradually increased, so that the moving speed of the syringe unit 20D can be gradually reduced.

A plurality of the deceleration portions 402 is provided along the circumferential direction of the syringe holder 70D. With this configuration, the moving speed of the syringe 68 when the syringe 68 moves in the distal direction can be stably reduced.

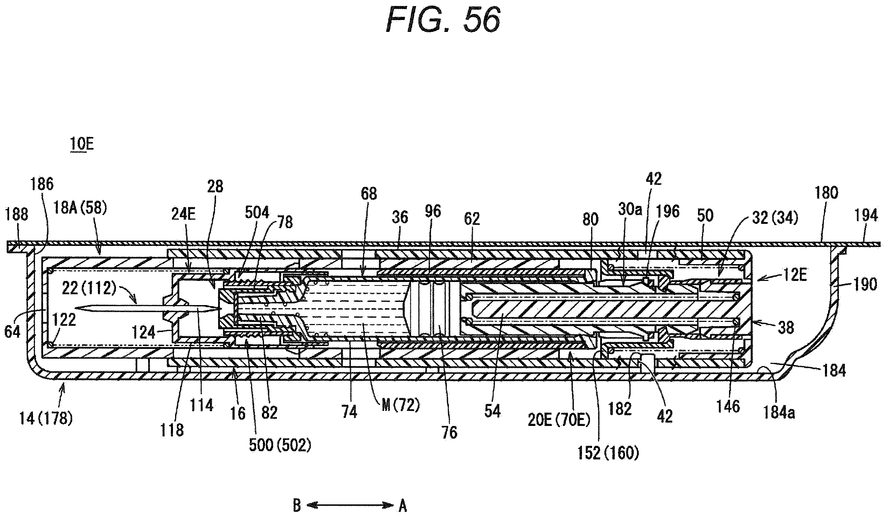

Next, a liquid medicine administration system 10E will be described with reference to Figs. 56 to 63. Note that the same components as those of the liquid medicine administration systems 10A, 10B, 10C, and 10D are denoted by the same reference numerals, and the detailed description thereof will be omitted.

The liquid medicine administration system 10E illustrated in Fig. 56 includes a liquid medicine administration device 12E and a packaging container 14 that accommodates the liquid medicine administration device 12E.

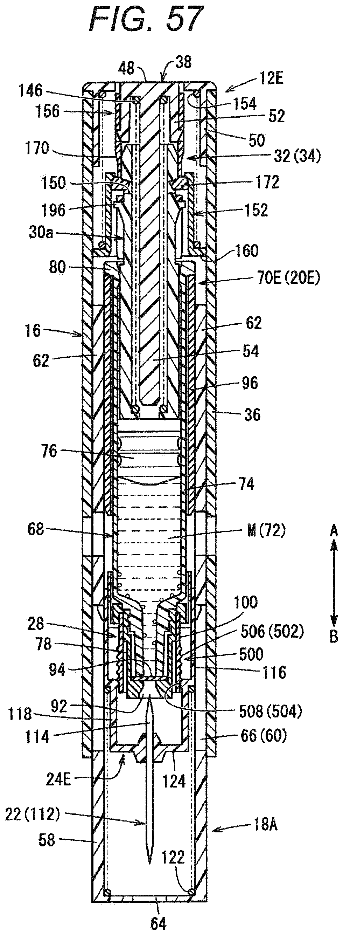

The liquid medicine administration device 12E illustrated in Fig. 57 includes a vibration generating mechanism 500 capable of applying vibration to the syringe unit 20E when the syringe unit 20E moves in the distal direction along a housing 16.

The vibration generating mechanism 500 includes a first vibration generating portion 502 provided on the outer peripheral surface of the syringe unit 20E and a second vibration generating portion 504 formed on a needle hub 24E fixed inside the housing 16, the second vibration generating portion 504 being capable of coming into contact with the first vibration generating portion 502 when the syringe unit 20E moves in the distal direction.

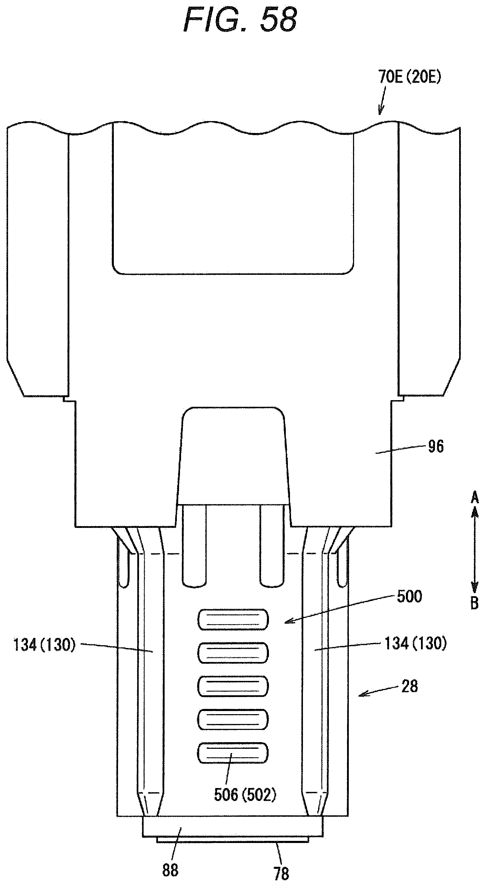

As illustrated in Figs. 57 and 58, the first vibration generating portion 502 is disposed on the outer peripheral surface of a holder distal cylindrical portion 100 which is the distal portion of a syringe holder 70E. The first vibration generating portion 502 includes a first protrusion 506 protruding radially outward from the outer peripheral surface of the holder distal cylindrical portion 100. Note that the first vibration generating portion 502 is provided on the outer peripheral surface of a syringe 68 in a liquid medicine administration device including only the syringe 68 without the syringe holder 70E.

A plurality of the first protrusions 506 is provided along a moving direction (direction of arrow A, B) of the syringe unit 20E, and the first protrusions 506 are spaced apart from each other in the moving direction. A pair of the first protrusions 506 is disposed in the circumferential direction of the syringe unit 20E. The first protrusion 506 has a triangular cross-sectional shape tapered radially outward from the outer peripheral surface of the syringe 68. The first protrusion 506 may have an annular shape extending along the circumferential direction of the syringe unit 20E.

As illustrated in Fig. 57, the second vibration generating portion 504 is provided on the inner peripheral surface of the proximal portion of a guide cylindrical portion 118 of the needle hub 24E and has a second protrusion 508 protruding radially inward from the inner peripheral surface. The second protrusion 508 is disposed at a position facing the first protrusion 506. The second protrusion 508 has a triangular cross-sectional shape tapered radially inward from the inner peripheral surface of the needle hub 24E. As viewed in the axial direction of the syringe 68, the first protrusion 506 and the second protrusion 508 are disposed to overlap each other.

A single second protrusion 508 may be provided, or a plurality of second protrusions 508 may be provided along the moving direction of the syringe unit 20E. When the plurality of second protrusions 508 is provided, the second protrusions 508 are spaced from each other in the axial direction of the needle hub 24E.

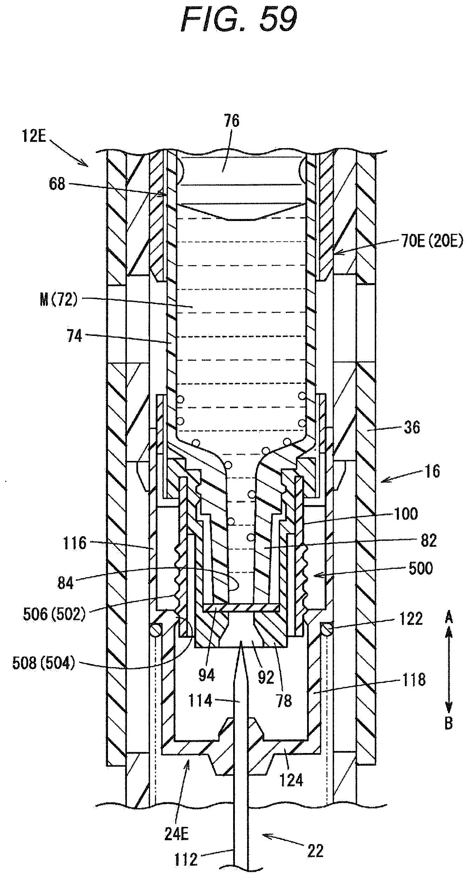

In the liquid medicine administration device 12E, during the process of the puncture operation, the syringe unit 20E moves in the distal direction together with a plunger 30 by the resilient force of an injection spring 146 from an initial state illustrated in Fig. 57. Then, as illustrated in Fig. 59, the distal end of the holder distal cylindrical portion 100 is inserted into the proximal end of the guide cylindrical portion 118 of the needle hub 24E, and before a second needle portion 114 is connected to the distal end (lid body 94) of the syringe 68, the distal-most first protrusion 506 among the plurality of first protrusions 506 comes into contact with the second protrusion 508. Vibration is generated along with the contact between the first protrusion 506 and the second protrusion 508, and the vibration is applied to the vicinity of the nozzle 82 which is the distal end of the syringe 68.

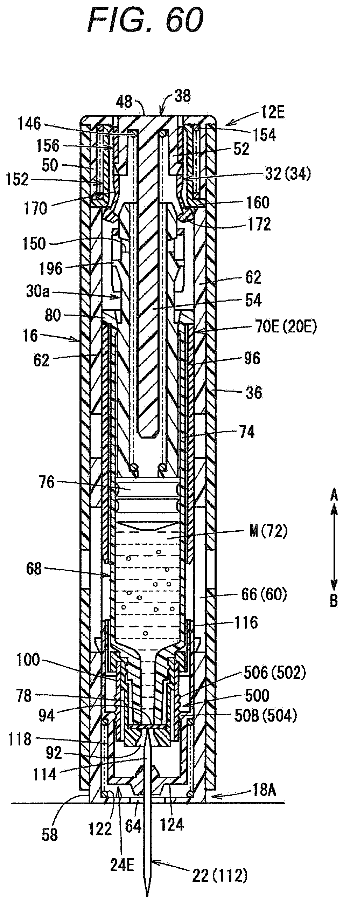

The liquid medicine administration device 12E is usually used with the distal end facing downward or obliquely downward. Therefore, due to vibration applied to the vicinity of the nozzle 82 of the syringe 68 by the vibration generating mechanism 500, air bubbles (air) contained in a liquid medicine M in a medicine chamber 72 of a barrel 74 are separated from the inner wall of the barrel 74, and can be moved toward a gasket 76 located above by the buoyancy of the air bubbles (see Fig. 60).

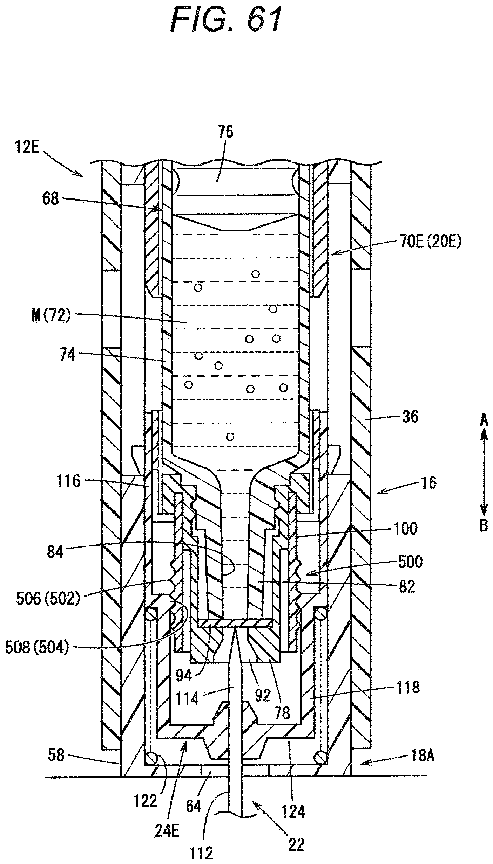

As illustrated in Fig. 61, with the movement of the syringe unit 20E in the distal direction, the plurality of first protrusions 506 is continuously brought into contact with the second protrusion 508, so that vibration is applied to the vicinity of the nozzle 82 of the syringe 68 a plurality of times. Therefore, air bubbles contained in the liquid medicine M can be reliably separated from the inner wall of the barrel 74 and can be moved toward the gasket 76.

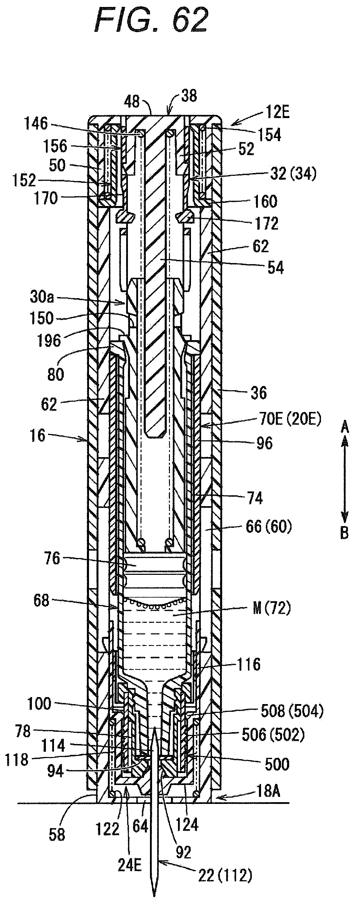

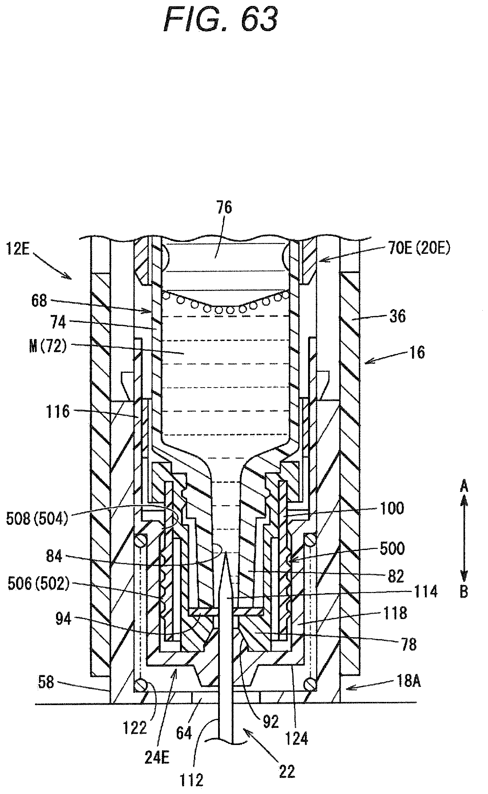

As illustrated in Fig. 62, after the proximal-most first protrusion 506 climbs over the second protrusion 508 in the distal direction, the distal end of the syringe unit 20E comes into contact with a support portion 124 of the needle hub 24E, whereby the movement of the syringe unit 20E is stopped. The connection between the syringe unit 20E and the second needle portion 114 is completed (see Fig. 63).

The plunger 30 further moves in the distal direction by the resilient force of the injection spring 146, and the plunger 30 pushes the gasket 76 in the distal direction, so that the liquid medicine M is administered to the subcutaneous part of the user through lumens 112a and 114a of a double-ended needle 22. At this time, as illustrated in Figs. 62 and 63, the air bubbles in the liquid medicine M have moved toward the gasket 76 (direction of arrow A) opposite to the double-ended needle 22 by the vibration generated by the vibration generating mechanism 500. Therefore, it is possible to administer only the liquid medicine M not containing air bubbles through the double-ended needle 22.

In addition, in a case where the liquid medicine M is quantitatively administered using the plunger 30a having the projections 196 illustrated in Fig. 34, the liquid medicine M can be accurately administered in a desired quantitative amount by moving air bubbles in the proximal direction using the vibration generating mechanism 500. This is effective.

The needle of the liquid medicine administration device 12E having the vibration generating mechanism 500 is not limited to the double-ended needle 22 having the first and second needle portions 112 and 114 and connected to the distal end along with the movement of the syringe unit 20E. The needle may be a single-ended needle connected in advance to the distal end of the syringe 68.

As described above, the liquid medicine administration device 12E includes the vibration generating mechanism 500 capable of applying vibration to the syringe unit 20E when the syringe unit 20E moves in the distal direction along the housing 16 during the puncture operation. The application of vibration to the syringe unit 20E by the vibration generating mechanism 500 can separate air bubbles contained in the liquid medicine M filled in the barrel 74 of the syringe 68 from the inner wall of the barrel 74 and can move the air bubbles toward the gasket 76 located above by the buoyancy of the air bubbles. As a result, when the liquid medicine M is administered into the subcutaneous part of the user through the double-ended needle 22, air bubbles contained in the administered liquid medicine M can be reduced, and the liquid medicine M can be accurately administered to the subcutaneous part in a predetermined amount.

The vibration generating mechanism 500 includes the first vibration generating portion 502 provided on the outer peripheral surface of the syringe unit 20E and the second vibration generating portion 504 provided on the inner peripheral surface of the needle hub 24E and capable of coming into contact with the first vibration generating portion 502 when the syringe unit 20E moves in the distal direction. With this configuration, vibration can be easily and reliably generated using the movement of the syringe unit 20E in the distal direction without providing a complicated mechanism such as an actuator.

The number of at least one of the first vibration generating portion 502 and the second vibration generating portion 504 is at least two. With this configuration, vibration can be generated a plurality of times when the syringe unit 20E moves in the distal direction, whereby air bubbles contained in the liquid medicine M can be more reliably moved upward in the barrel 74.

The first vibration generating portion 502 includes the first protrusion 506 protruding radially outward from the outer peripheral surface of the holder distal cylindrical portion 100 of the syringe holder 70E and the second vibration generating portion 504 includes the second protrusion 508 protruding radially inward from the inner peripheral surface of the guide cylindrical portion 118 of the needle hub 24E. With this configuration, when the syringe unit 20E is moved toward the distal end, vibration can be intermittently applied to the syringe unit 20E by bringing the first protrusion 506 and the second protrusion 508 into contact with each other. Therefore, the air bubbles contained in the liquid medicine M and attached to the inner wall of the barrel 74 can be reliably separated from the inner wall and moved upward.

The first vibration generating portion 502 is disposed on the holder distal cylindrical portion 100 that is the distal end of the syringe holder 70E. With this configuration, it is possible to reliably separate air bubbles in the liquid medicine M that are likely to accumulate at the distal portion in the barrel 74 of the syringe 68 from the inner wall of the barrel 74 and to move the air bubbles in the proximal direction from the distal portion of the syringe 68.

The above-described embodiments are summarized as follows.

A liquid medicine administration device (12A) according to the above embodiments includes:
a housing (16) formed in a hollow cylindrical shape;
a syringe (68) that includes a barrel (74) filled with a liquid medicine (M) and is disposed within the housing;
a syringe holder (70A) that accommodates the syringe, holds the syringe, and is disposed within the housing so as to be movable in an axial direction of the housing; and
a double-ended needle (22) including a first needle portion (112) that protrudes in a distal direction and punctures a puncture site, and a second needle portion (114) that protrudes in a proximal direction, wherein
the syringe holder includes a holding portion (106, 106a) that holds a distal portion of the syringe coaxially with a distal portion of the syringe holder by elastically biasing the distal portion of the syringe radially inward.

The holding portion has a plurality of flexible arms (108, 108a) that presses an outer peripheral surface of the distal portion of the syringe radially inward at a plurality of positions in a circumferential direction.

The plurality of flexible arms is arranged at equal intervals along the circumferential direction of the syringe holder.

The syringe includes a cap (78) attached to a distal end of the barrel, and
the plurality of flexible arms presses the cap.

The syringe holder includes a holder body (96) that accommodates the barrel, and a holder distal cylindrical portion (100) that is reduced in diameter than the holder body and extends from a distal end of the holder body in the distal direction, and
the plurality of flexible arms (108) protrudes in the distal direction from the distal end of the holder body, and inner ends of the flexible arms in a radial direction are in contact with an outer peripheral surface of the distal portion of the syringe.

The syringe holder includes a holder body that accommodates the barrel, and a holder distal cylindrical portion that is reduced in diameter than the holder body and extends from a distal end of the holder body in the distal direction, and
the plurality of flexible arms (108a) protrudes in the proximal direction from a proximal end of the holder distal cylindrical portion, and inner surfaces of the flexible arms are in contact with an outer peripheral surface of the distal portion of the syringe.

The cap has a stepped portion (90) provided on an outer peripheral surface of the cap between a distal end and a proximal end of the cap, and
the plurality of flexible arms is engaged with a distal side of the stepped portion.

Note that the present invention is not limited to the above disclosure, and various configurations may be adopted without departing from the gist of the present invention.

## Claims

1. A liquid medicine administration device comprising:
a housing formed in a hollow cylindrical shape;
a syringe that includes a barrel filled with a liquid medicine and is disposed within the housing;
a syringe holder that accommodates the syringe, holds the syringe, and is disposed within the housing so as to be movable in an axial direction of the housing; and
a double-ended needle including a first needle portion that protrudes in a distal direction and punctures a puncture site, and a second needle portion that protrudes in a proximal direction, wherein
the syringe holder includes a holding portion that holds a distal portion of the syringe coaxially with a distal portion of the syringe holder by elastically biasing the distal portion of the syringe radially inward.

2. The liquid medicine administration device according to claim 1, wherein
the holding portion has a plurality of flexible arms that presses an outer peripheral surface of the distal portion of the syringe radially inward at a plurality of positions in a circumferential direction.

3. The liquid medicine administration device according to claim 2, wherein
the plurality of flexible arms is arranged at equal intervals along the circumferential direction of the syringe holder.

4. The liquid medicine administration device according to claim 2 or 3, wherein
the syringe includes a cap attached to a distal end of the barrel, and
the plurality of flexible arms presses the cap.

5. The liquid medicine administration device according to any one of claims 2 to 4, wherein
the syringe holder includes a holder body that accommodates the barrel, and a holder distal cylindrical portion that is reduced in diameter than the holder body and extends from a distal end of the holder body in the distal direction, and
the plurality of flexible arms protrudes in the distal direction from the distal end of the holder body, and inner ends of the flexible arms in a radial direction are in contact with an outer peripheral surface of the distal portion of the syringe.

6. The liquid medicine administration device according to any one of claims 2 to 4, wherein
the syringe holder includes a holder body that accommodates the barrel, and a holder distal cylindrical portion that is reduced in diameter than the holder body and extends from a distal end of the holder body in the distal direction, and
the flexible arms protrudes in the proximal direction from a proximal end of the holder distal cylindrical portion, and inner surfaces of the flexible arms are in contact with an outer peripheral surface of the distal portion of the syringe.

7. The liquid medicine administration device according to claim 4, wherein
the cap has a stepped portion provided on an outer peripheral surface of the cap between a distal end and a proximal end of the cap, and
the plurality of flexible arms is engaged with a distal side of the stepped portion.
